# EUROPEAN PATENT SPECIFICATION

(11) **EP 4 381 291 B1**
(45) Date of publication and mention of the grant of the patent: **31.12.2025**
(21) Application number: 22761219.9
(22) Date of filing: 05.08.2022
(51) Int. Cl.: G01N 33/50, G01N 33/53, G01N 33/58

(54) **STANDARD FOR GLYCOPROFILING OF PROTEINS**
STANDARD ZUR GLYCOPROFILIERUNG VON PROTEINEN
NORME POUR LE GLYCOPROFILAGE DE PROTÉINES

(30) Priority: 06.08.2021 EP 21190083
(43) Date of publication of application: 12.06.2024
(73) Proprietor: Glycanostics s.r.o., 841 01 Bratislava (SK)
(72) Inventor: TKAC, Jan, 841 01 Bratislava mestská cast Dúbravka (SK); BERTOK, Tomas, 841 01 Bratislava mestská cast Dúbravka (SK)
(74) Representative: Schiweck Weinzierl Koch Patentanwälte Partnerschaft mbB
(86) International application number: PCT/EP2022/072138
(87) International publication number: WO 2023/012352

(56) References cited:
- US-A1- 2014 005 069
- KUBERAN B ET AL: "PREPARATION AND ISOLATION OF NEOGLYCOCONJUGATES USING BIOTIN-STREPTAVIDIN COMPLEXES", GLYCOCONJUGATE JOURNAL, CHAPMAN & HALL, BOSTON, vol. 16, 1 January 1999 (1999-01-01), pages 271 - 281, XP002927347, ISSN: 0282-0080, DOI: 10.1023/A:1007009927087
- SHAO MING-CHUAN ET AL: "The use of avidin-biotinylglycan as the model for in vitro glycoprotein processing", JOURNAL OF BIOLOGICAL CHEMISTRY, AMERICAN SOCIETY FOR BIOCHEMISTRY AND MOLECULAR BIOLOGY, US, vol. 262, no. 7, 1 January 1987 (1987-01-01), pages 2968 - 2972, XP002470272, ISSN: 0021-9258

## Description

### TECHNICAL FIELD OF THE INVENTION

The present invention relates to a method of relativizing signals by using a neoglycoprotein as a standard for glycoproteins, wherein said neoglycoprotein comprises a streptavidin molecule bound through biotin to at least one, preferably four, pre-defined glycan determinant(s) which comprises said glycan structure (A) and a corresponding use of said neoglycoprotein. Thereby, statements whether a particular glycan structure (A) is present on a protein of interest are possible. Applications in diagnosis of diseases such as cancer, autoimmune disease or inflammatory disease are disclosed.

### BACKGROUND

Glycans are present on a variety of different proteins, where they have an impact on protein trafficking, stability and folding, ultimately altering its biochemical, and biophysical properties. Moreover, glycans can mediate proteolysis patterns or directly mediate ligand-receptor interactions, oncogenic signaling transduction, immune recognition, migration and both cell-cell and cell-matrix adhesion. As such, particular glycans may exert a selective advantage for tumor cells. The presence of particular glycans or the presence of particular glycans on particular proteins thus may be used as a biomarker, e.g., for the diagnosis of cancer.

Glycan structures can be analyzed by using binding molecules that specifically bind to a particular glycan structure. US 2014/005069; Kuberan et al. 1999, Glycoconjugate Journal, Chapman & Hall, Boston, vol. 16; and Shao Ming-Chuan et al. 1987, American Society for Biochemistry and Molecular Biology, vol. 262, no. 7 disclose the determination of glycan structures present on proteins of interest, comprising comparing the signals obtained from determining said glycan structures present on said proteins of interest with the signals obtained from determining said glycan structures present on neoglycoprotein which consist of glycan structures bound through biotin to streptavidin molecules. Besides antibodies specific for glycan structures also lectins can be employed. Lectins are carbohydrate-binding proteins that are highly specific for sugar groups that are part of other molecules. These binding molecules can be used in assays like enzyme-linked immunosorbent assay (ELISA), enzyme-linked lectin assay (ELLA), magnetic ELLA (MELLA) to analyze the presence or absence of a particular glycan structure.

The signals obtained by applying these methods however needs to be relativized by a (positive) control or standard to allow valid statements whether there is said particular glycan structure present or not or to quantify it in a sample. Commercially available glycoprotein standards contain only one glycan structure, which is not identical to the glycan structure of interest, i.e. these cannot be used for assessing whether a glycan structure relevant for a disease is present on the protein of interest or not. Thus, protein standards comprising a particular glycan structure are not easily available. One possibility to obtain such controls is to conjugate the glycan structure of interest to the protein of interest. However, this is complicated, labor-intensive and expensive. Furthermore, it has to be repeated for every new combination of glycan structure of interest and protein of interest.

Consequently, there is an ongoing need for reliable, inexpensive and versatile glycoprotein standards. The present invention aims to address this need.

### SUMMARY OF THE INVENTION

This need is solved by the subject-matter as defined in the claims and in the embodiments described herein.

The inventors surprisingly found that it is not necessary to provide such a modified (glyco)protein of interest to relativize a signal obtained from determining a signal (1) obtained from determining a glycan structure (A) suspected to be present on a protein of interest but instead a neoglycoprotein can be used as a standard. This standard provides the signal (2) obtained from determining said glycan structure (A) actually comprised by a neoglycoprotein. The neoglycoprotein comprises a streptavidin molecule bound through biotin to at least one pre-defined glycan determinant which comprises said glycan structure (A). Thus, streptavidin can be seen as the scaffold for a neoglycoprotein, which itself acts as a standard for relativizing. As shown in the Examples, streptavidin loaded with biotinylated glycan structures (A) can be used as a standard.

The present invention is set out in the appended set of claims.

### BRIEF DESCRIPTION OF THE DRAWINGS

The invention will be better understood with reference to the detailed description when considered in conjunction with the non-limiting examples and the accompanying drawings, in which:

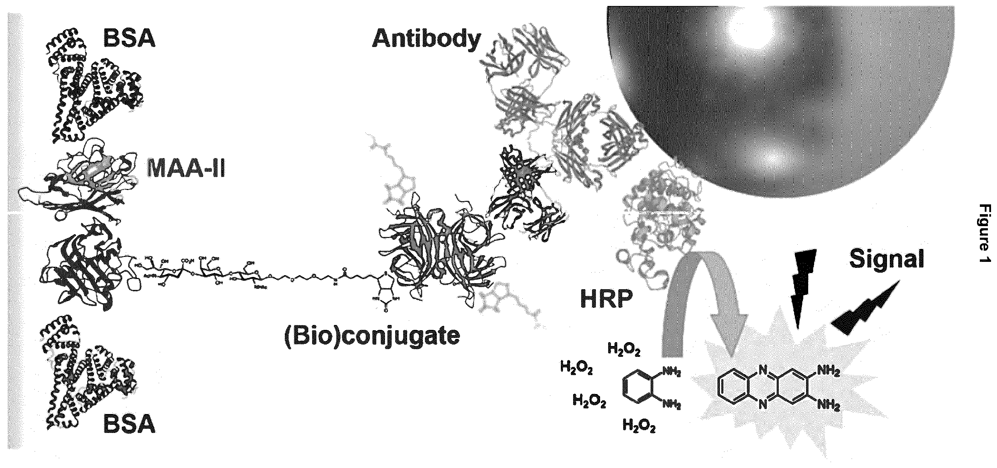

**Figure 1** depicts an exemplary scheme of an exemplary embodiment of the invention. MAA-II lectin and optionally a blocking agent such as BSA are physically adsorbed on the bottom of an ELISA plate well. A magnetic particle with co-immobilized anti-streptavidin antibody and horseradish peroxidase (HRP) is used to selectively fish out an analyte (glycan-streptavidin bioconjugate in this case) and is subsequently applied to lectin-biorecognition interface. Optical signal is generated using o-phenylenediamine and hydrogen peroxide to form a coloured product 2,3-diaminophenazine, detected by a common ELISA reader (e.g., λ = 450-490 nm).
**Figure 2A** depicts the slope values during pH scouting for streptavidin (left columns) and MAA-II lectin (right columns). **Figure 2B** shows sensorgrams depicting immobilization of three different ligands in pH 4.0 acetate buffer using CM5 chip.
**Figure 3** depicts an SCK (single cycle kinetics) analysis of anti-streptavidin Ab on a streptavidin-modified CM5 chip.
**Figure 4** depicts a bare Au SPR chip (A) with a prism on one site, modified by (B) self-assembled monolayer of 11-mercaptoundecanoic acid or (C) carboxymethyl-dextran, creating 2D and 3D matrix, respectively. Since evanescent wave amplitude decays exponentially (red arrow), 3D matrix together with a high concentration of negative charge, steric obstacles and higher chance of re-binding during dissociation phase was less suitable to observe the preparation of a sandwich configuration, *i. e*. MAA-II/neoglycoprotein/Ab.
**Figure 5** depicts the assay workflow used for 2D configuration, including a schematic presentation of the surface after each step (**Fig. 5A**) and sensorgram of neoglycoprotein (glycoconjugate) capturing and binding analysis of anti-streptavidin antibody (**Fig. 5B**).
**Figure 6** depicts a model situation on the planar surface (e. g. SPR chip, **A**) when the linker density is (in the case of 2D configuration, *i.e.* no diffusion barrier in the matrix) the same near the Au surface and on the interface. The situation is, however, different for spherical interface (e. g. MNPs, **B**). NTA analysis of three samples showing successful immobilization and interaction with a neoglycoprotein (**C**).
**Figure 7** depicts an NTA analysis of unmodified MNPs (dark line) and MNPs + Ab (in case of excess of antibody was used, light line) - an obvious increase in peak maximum caused by antibody immobilized on the surface was observed.
**Figure 8** depicts an MS analysis of the neoglycoprotein (protein standard), showing the highest intensity for a peak at ~13 kDa (a single streptavidin monomer).

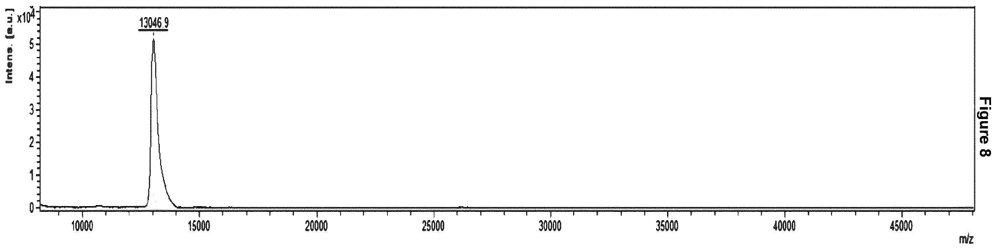

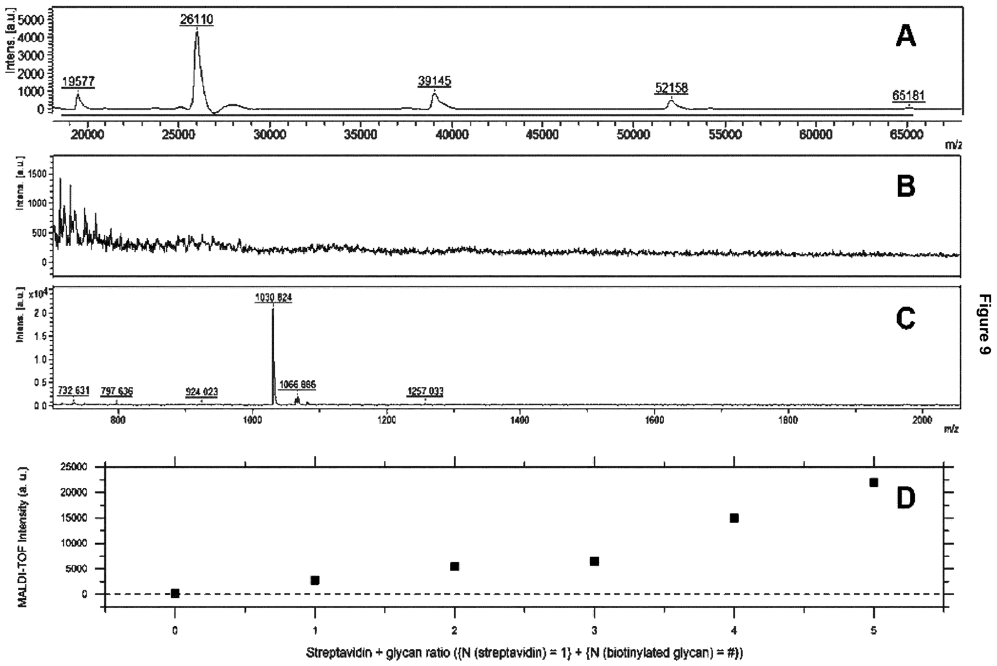

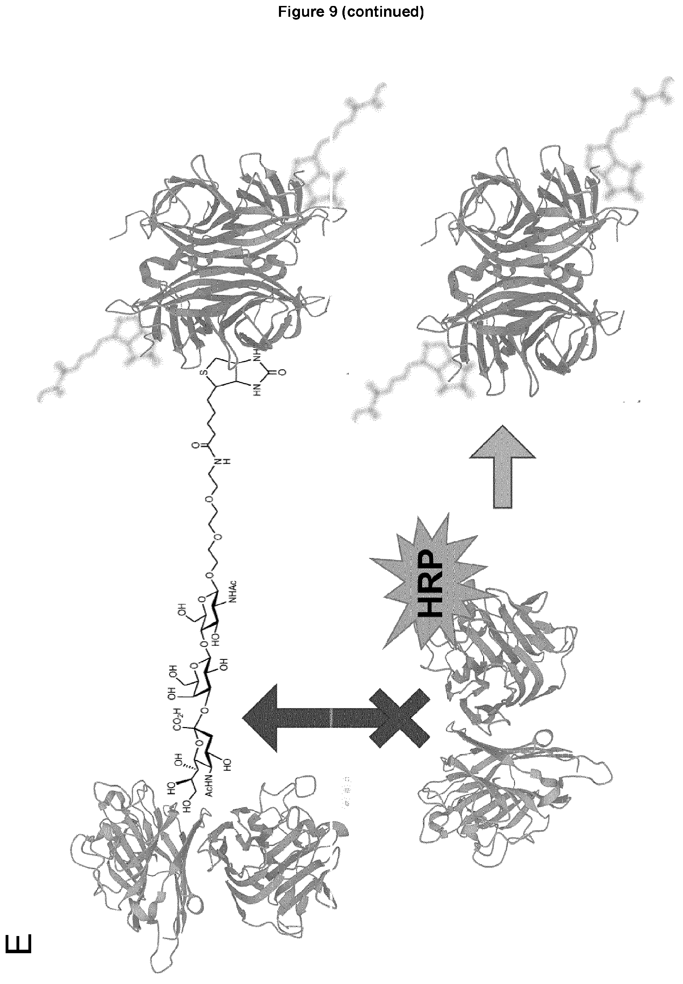

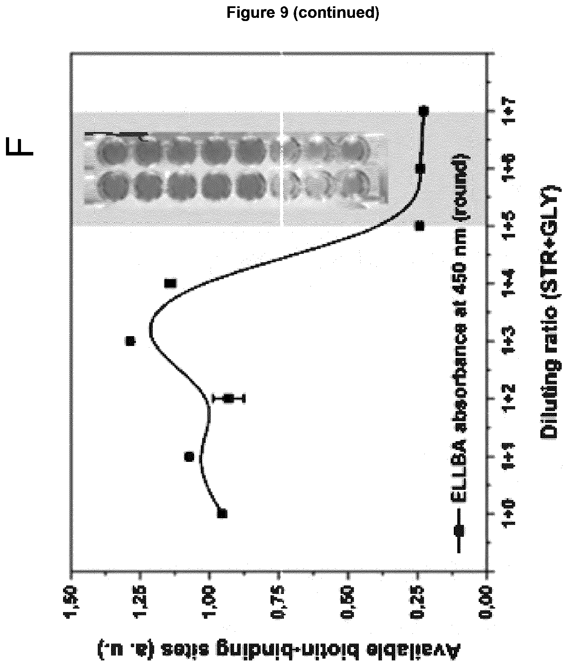

**Figure 9** depicts an MS analysis showing other peaks (with lower intensities compared to Fig. 8) (**A**); Peak of ~1030 Da, corresponding to a biotinylated glycan, cannot be detected in a sample with a pure streptavidin (**B**), however, is present in all the other samples - even with lower glycan/streptavidin ratio and after desalting procedure, implicating the glycan is present on the streptavidin (**C**) - a fact already confirmed using SPR. Intensity of this peak increases with the increasing glycan/streptavidin ratio (**D**). ELLBA was used to find the optimal ratio to prepare a neoglycoprotein with saturated density of a glycan in a competitive configuration using unconjugated and biotinylated MAA-II lectins (**E**), where ratio of 1+5 and higher proved to be optimal (**F**).

### DETAILED DESCRIPTION OF THE INVENTION

The present invention is described in detail in the following and will also be further illustrated by the appended examples and figures.

If available, the standards used in prior art typically are the proteins of interest, which are labelled with the glycan structure (A) (of interest), which is suspected to be on the protein of interest. The standard in prior art is thus the protein of interest itself, which comprises the glycan structure (A). Synthesizing such standards is however, complicated, time-consuming and expensive. The present invention thus aims at providing a reliable, inexpensive and versatile glycan structure or glycoprotein standard for relativizing signals obtained from determining glycan structures suspected to be present on a protein of interest.

The inventors surprisingly found that it is not necessary to provide such a modified (glyco)protein of interest to relativize a signal obtained from determining a signal (1) obtained from determining a glycan structure (A) suspected to be present on a protein of interest but instead a neoglycoprotein can be used as a standard. This signal (2) obtained from determining a glycan structure (A) on the neoglycoprotein allows the relativization of signal (1). The neoglycoprotein comprises a streptavidin molecule bound through biotin to at least one pre-defined glycan determinant which comprises said glycan structure (A). Thus, streptavidin can be seen as the scaffold for a neoglycoprotein, to which the glycan structure (A) of interest can be coupled.

As shown in the Examples, streptavidin loaded with biotinylated glycan structures (A) can be used as a standard. This is the first study to develop a glycoconjugate or an exemplary neoglycoprotein as described herein consisting of a streptavidin molecule bound to up to four biotinyl-glycans to form a neoglycoprotein with 4 glycans on the protein/streptavidin scaffold. At the same time the inventive concept allows preparing any kind of neoglycoprotein with a defined glycan structure (A) present on the surface of streptavidin using biotinylated glycans. Such a neoglycoprotein with (up to) 4 glycans may then be used as a protein standard for determining said glycan structure (A), e.g. with the MELLA technology disclosed in WO 2019/185515 A1. In an exemplary embodiment, such neoglycoproteins are able to bind to a lectin and an anti-streptavidin antibody at the same time, i.e. in a sandwich configuration to provide an optical signal, which is considered to be the signal to which signal from analysing samples can be relativized using Glycanostics's MELLA protocol for prostate cancer (among others) diagnostics (Fig. 1).

Accordingly, the present invention relates to a method for relativizing a signal (1) obtained from determining a glycan structure (A) suspected to be present on a protein of interest, comprising a) determining a glycan structure (A) suspected to be present on a protein of interest, which provides a signal (1); and b) comparing the signal (1) obtained from determining said glycan structure (A) suspected to be present on said protein of interest in step a) with a signal (2) obtained from determining said glycan structure (A) actually comprised by a neoglycoprotein, wherein said neoglycoprotein comprises a streptavidin molecule bound through biotin to at least one pre-defined glycan determinant which comprises said glycan structure (A), thereby relativizing said signal (1) to said signal (2), or vice versa.

Also, the present invention relates to a method for relativizing a signal (1) obtained from determining a glycan structure (A) suspected to be present on a protein of interest, comprising a) determining a glycan structure (A) suspected to be present on a protein of interest, which provides a signal (1); and b) comparing the signal (1) obtained from determining said glycan structure (A) suspected to be present on said protein of interest in step a) with a signal (2) obtained from determining said glycan structure (A) actually comprised by a neoglycoprotein acting as a standard, wherein said neoglycoprotein comprises a streptavidin molecule bound through biotin-streptavidin interaction to at least one pre-defined glycan determinant which comprises said glycan structure (A), wherein relativizing comprises comparing signal (1) with signal (2) from the standard, thereby signal (2) allows putting the information obtained by signal (1) in relation, thereby relativizing said signal (1) to said signal (2), or vice versa.

The present invention further relates to the use of a neoglycoprotein comprising a streptavidin molecule bound through biotin to at least one pre-defined glycan determinant which actually comprises a glycan structure (A) suspected to be present on a protein of interest for relativizing a signal (1) obtained from determining glycan structure (A) suspected to be present on a protein of interest to a signal (2) obtained from determining said glycan structure (A) actually comprised by said neoglycoprotein, wherein said neoglycoprotein comprises a streptavidin molecule bound through a streptavidin-binding molecule to at least one pre-defined glycan determinant which comprises said glycan structure (A).

Also, the present invention relates to the use of a neoglycoprotein acting as a standard comprising a streptavidin molecule bound through biotin to at least one pre-defined glycan determinant which actually comprises a glycan structure (A) suspected to be present on a protein of interest for relativizing a signal (1) obtained from determining glycan structure (A) suspected to be present on a protein of interest to a signal (2) obtained from determining said glycan structure (A) actually comprised by said neoglycoprotein, wherein said neoglycoprotein comprises a streptavidin molecule bound through biotin-streptavidin interaction to at least one pre-defined glycan determinant which comprises said glycan structure (A) wherein relativizing comprises comparing signal (1) with signal (2) from the standard, thereby signal (2) allows putting the information obtained by signal (1) in relation

"Relativizing" can be seen as describing the process of comparing one signal (1) with another signal (2), thereby providing information on how signal (1) relates to signal (2). Signal (2) can be seen as the standard in the context of the invention. In other words, signal (2) allows putting the information obtained by signal (1), e.g., the signal intensity, in context or relation. Thus, signal (2) may act as a positive control, thereby providing information on the signal that is needed to consider signal (1) as positive. Alternatively or additionally, signal (2) may not only be a positive control but also be the result of a concentration series, thereby allowing the quantification of the amount of the glycan structure (A) by comparing (relativizing) signal (1) to a series of signals (2) at different concentrations. In this context, the methods and uses of the invention can be also described as glycoprofiling the protein of interest.

In line with the above, "relativizing" may comprise comparing signal (1) with signal (2) from the standard, thereby signal (2) allows putting the information obtained by signal (1) in relation. Advantageously, with such a comparison, signal (1) is relativized to said signal (2), or vice versa.

The term "glycoprofile of a protein" means a carbohydrate structure of the protein of interest, e.g., composition and/or structure of covalently linked carbohydrates, e.g., quantity, presence, or absence of covalently linked carbohydrates. The term "glycoprofiling" means determining a carbohydrate structure (e.g., composition and/or structure of covalently linked carbohydrates, e.g., quantity, presence, or absence of covalently linked carbohydrates) on a protein of interest.

The information provided by signal (1) and signal (2) can provide the information whether the glycan structure (A) is present on the protein of interest or not. For this purpose, signal (1) (obtained from determining said glycan structure (A) suspected to be present on said protein of interest) is compared (relativized) with the standard/signal (2) (obtained from determining said glycan structure (A) actually comprised by said neoglycoprotein). In case signal (1) is equal or higher than signal (2), it is indicative that said glycan structure (A) is considered to be present on the protein of interest. Accordingly, relativizing may comprise comparing the signal (1) obtained from determining said glycan structure (A) suspected to be present on said protein of interest with a signal (2) obtained from determining said glycan structure (A) actually comprised by said neoglycoprotein, wherein, if signal (1) is lower than signal (2), it is indicative that said suspected glycan structure (A) is not present on said protein of interest.

Otherwise, if signal (1) is lower than signal (2), it is indicative that said glycan structure (A) is considered not to be present on the protein of interest. Accordingly, relativizing may comprise comparing the signal (1) obtained from determining said glycan structure (A) suspected to be present on said protein of interest with a signal (2) obtained from determining said glycan structure (A) actually comprised by said neoglycoprotein, wherein, if signal (1) is equal to or higher than signal (2), it is indicative that said suspected glycan structure (A) is present on said protein of interest.

The methods and uses of the present invention however are not limited to qualitative statements. In contrast, if signal (1) is compared to a series of signals (2), which are determined at a series of different concentrations of the neoglycoprotein, allow a quantitative analysis of the amount of glycan structures (A) suspected to be on the protein of interest in a sample. Accordingly, relativizing (in the methods and uses of the invention) may comprise comparing the signal (1) obtained from determining said glycan structure (A) suspected to be present on said protein of interest with the signal (2) obtained from determining a concentration series of said glycan structure (A) actually comprised by said neoglycoprotein.

A "concentration series" as described herein relates to data set of signals (2) obtained from determining the glycan structure (A) at two or more different concentrations of the neoglycoprotein. The data set may be used to provide or calculate a standard curve, e.g. by linear regression, that allows conclusions from the signal (intensity) (1) to the actual level or amount of the glycan structure (A) in a sample. The concentration series may also allow setting a predetermined threshold that provides for the signal (intensity), at which the glycan structure (A) is deemed to be present on the protein of interest. To put it differently, the concentration series may be used to set the baseline (threshold) for a data point, at which no neoglycoprotein has been added to the sample to be analyzed (in the concentration series). Accordingly, the concentration series may also comprise a concentration which corresponds to a predetermined threshold concentration above which said glycan structure (A) is known to be present on said protein of interest.

A "neoglycoprotein" as used herein relates to a streptavidin molecule, to which at least 1 and up to 4, i.e., 1, 2, 3 or 4, preferably 4, biotinylated glycan structures (A) are non-covalently bound by the biotin-streptavidin interaction. Accordingly, the neoglycoprotein is bound through biotin-streptavidin interaction. Streptavidin thereby acts as scaffold, which can be coupled to the desired glycan structures (A). Thereby, a highly versatile and quickly available neoglycoprotein standard can be provided. Accordingly, the glycan structure (A) is added to the streptavidin preferably in excess, e.g., at a molar ratio of at least 4:1 (glycan structure (A):streptavidin), at least 5:1, at least 7.5:1 or at least 10:1. Preferably, the glycan structure (A) is added to the streptavidin at a molar ratio of between 4.5:1 and 5.5:1, most preferably at a molar ratio of 5:1.

"Streptavidin" is a protein purified from the bacterium *Streptomyces avidinii.* Streptavidin homo-tetramers have an extraordinarily high affinity for biotin (also known as vitamin B7 or vitamin H). With a dissociation constant (K_{d}) on the order of around 10⁻¹⁴ mol/L, the binding of biotin to streptavidin is one of the strongest non-covalent interactions known in nature. An exemplary amino acid sequence of a wild type streptavidin is: MRKIVVAAIAVSLTTVSITASASADPSKDSKAQVSAAEAGITGTWYNQLGSTFIVTAGADGALTG TYESAVGNAESRYVLTGRYDSAPATDGSGTALGWTVAWKNNYRNAHSATTWSGQYVGGAEA RINTQWLLTSGTTEANAWKSTLVGHDTFTKVKPSAASIDAAKKAGVNNGNPLDAVQQ (SEQ ID NO: 1). An exemplary wild type sequence of streptavidin is also shown in UniProt database entry P22629, version 1 of 1 August 1991. Streptavidin as used herein, e.g., in the context of the methods or uses described herein, may also encompass streptavidin muteins. Streptavidin muteins are, e.g., disclosed in WO 2017/186669 or WO 2014/076277. Streptavidin or streptavidin muteins used in the methods and uses of the invention may be derived from streptavidin variants which are shortened at the N- or/and the C-terminus. A preferred polypeptide according to the present invention comprises the amino acid sequence of a minimal streptavidin which begins N-terminally in the region of the amino acid positions 10 to 16 and terminates C-terminally in the region of the amino acid positions 133 to 142. Such a streptavidin mutein polypeptide corresponds preferably to a minimal streptavidin outside of the mutation region which comprises an amino acid sequence from position Ala13 to Ser139 and optionally has an N-terminal methionine residue instead of Ala13. In this application the numbering of amino acid positions refers throughout to the numbering of mature wt-streptavidin (Argarana et al., Nucleic Acids Res. 14 (1986), 1871-1882, cf. SEQ ID NO: 1) which is also deposited under accession number UniProtKB - P22629, v1 of 1 August 1991. "Streptavidin" as used here, in the context of the methods or uses described herein, may also relate to other biotin-binding moieties besides streptavidin, e.g. proteins or aptamers binding to biotin.

Streptavidin may have an amino acid sequence that is substantially identical to that of SEQ ID NO: 1. Streptavidin as used herein may have an amino acid sequence having a sequence identity of at least 80%, at least 90%, at least 95%, at least 98%, at least 99% or 100% to of SEQ ID NO: 1. Streptavidin as used herein may consist of an amino acid sequence having a sequence identity of at least 80%, at least 90%, at least 95%, at least 98%, at least 99% or 100% to of SEQ ID NO: 1.

Generally, when used herein, the terms "percent (%) identical" or "percent (%) identity," in the context of two or more nucleic acids or polypeptide sequences, refers to the extent to which two or more sequences or subsequences that are the same. Two sequences are "identical" if they have the same sequence of amino acids or nucleotides over the region being compared. Two sequences are "substantially identical" if two sequences have a specified percentage of amino acid residues or nucleotides that are the same (i.e., 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 30 nucleotides (or 10 amino acids) in length, or more preferably over a region that is 100 to 500 or 1000 or more nucleotides (or 20, 50, 200 or more amino acids) in length.

The percentage of sequence homology or sequence identity can, for example, be determined herein using the program BLASTP, version blastp 2.2.5 (November 16, 2002) (cf. Altschul et al., Nucleic Acids Res, 1997). In this embodiment the percentage of homology is based on the alignment of the entire polypeptide sequence (matrix: BLOSUM 62; gap costs: 11.1; cutoff value set to 10⁻³) including the propeptide sequences, preferably using the wild-type protein scaffold as reference in a pairwise comparison. It is calculated as the percentage of numbers of "positives" (homologous amino acids) indicated as result in the BLASTP program output divided by the total number of amino acids selected by the program for the alignment.

A "glycan" or a "glycan structure (A)" relates to compounds consisting of monosaccharides linked glycosidically and may also refer to carbohydrate portion of a glycoconjugate, such as a glycoprotein, glycolipid, glycoRNA (see, e.g., Flynn et al, Cell, 184(12):3109-3124) or a proteoglycan, even if the carbohydrate is only a monosaccharide or an oligosaccharide.

The glycan structure (A) bound to the streptavidin in the neoglycoprotein described herein is advantageously biotinylated. "Biotinylation" is the process of covalently attaching biotin to a protein, nucleic acid or other molecule, in particular to a glycan structure (A) as described herein. Various methods for biotinylating glycan structures (A) are known to a person skilled in the art. E.g., the biotinylation may be based on reductive amination in which a primary amine is coupled to an aldehyde to form an imine or a hydrazone if the primary amine is present as a hydrazide group. This imine (hydrazone) is then reduced to a secondary amine, which stabilizes the formed linkage (see scheme below). Using this reaction procedure, biotin-LC-hydrazide can be readily coupled to the reducing end of any carbohydrate to form a stable biotin-labeled product. See, e.g., Grün et al. (2006), Analytical Biochemistry, 354(1):54-63. 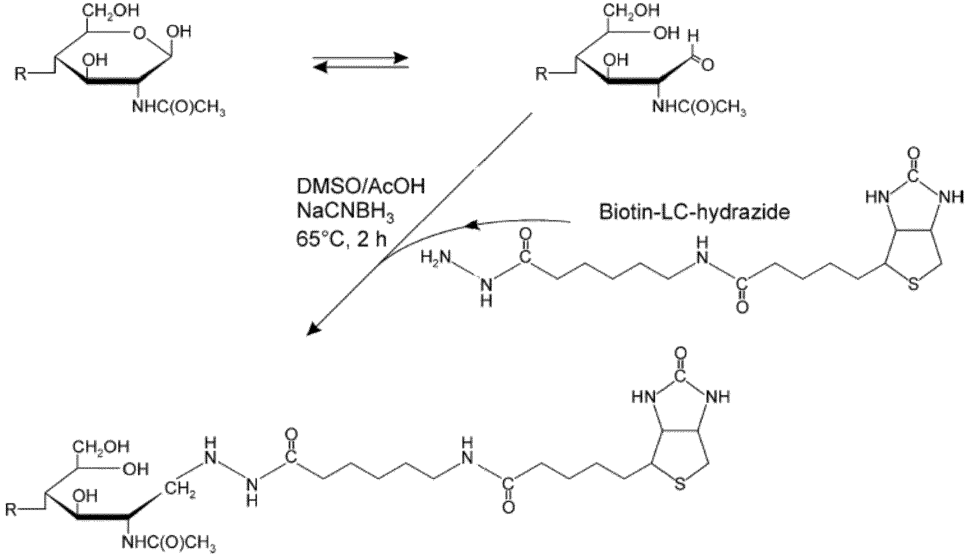 The biotin may also be coupled to the glycan by a polyethylene glycol linker such as a triethylene glycol (PEG3) spacer between glycan and biotin. An exemplary glycan structure (A) is 3'-sialyllactosamine-PEG3-biotin (single arm, ~1100 Da), a biotinylated 3'-Sialylated N-acetyllactosamine (LacNAc = LN = Galβ1,4GlcNAc) with beta-linked triethylene glycol (PEG3) spacer between glycan and biotin. Such biotinylated glycans are commercially available, e.g., from Sussex Chemicals, Ottawa, Canada.

The present invention is not limited to any particular glycan structure (A). In contrast, the modulary construction of the neoglycoprotein allows the non-covalent attachment of virtually any glycan structure (A) to the streptavidin, which is the scaffold of the neoglycoprotein described herein. Exemplary glycan structures (A) include, but are not limited to, core fucose, antennary fucose, Fucα1-6GlcNAc-*N*-Asn containing N-linked oligosaccharides, Fucα1-6/3GlcNAc, α-L-Fuc, Fucα1-2Galβ1-4(Fucα1-3)GlcNAc, Fucα1-2Gal, Fucα1-6GlcNAc, Manβ1-4GlcNAcβ1-4GIcNAc, branched N-linked hexa-saccharide, Manα1-3Man, α-D-Man, (GlcNAcβ1-4)₂₋₄, Galβ1-4GIcNAc, GlcNAcα1-4Galβ1-4GlcNAc, (GlcNAcβ1-4)₂₋₅, Neu5Ac (sialic acid), Galβ1-3GalNAc-serine/threonine, Galα1-3GalNAc, Galβ1-6Gal, Galβ1-4GlcNAc, Galβ1-3GalNAc, GalNAcα1-3GaINAc, GalNAcα1-3Gal, GalNAcα/β1-3/4Gal, α-GalNAc, GalNAcβ1-4Gal, GalNAcα1-3(Fucα1-2)Gal, GalNAcα1-2Gal, GalNAcα1-3GalNAc, GalNAcβ1-3/4Gal, GalNAc-Ser/Thr (Tn antigen), Galβ1-3GalNAc-Ser/Thr (T antigen), GalNAcβ1-4GlcNAc (LacdiNAc), α-2,3Neu5Ac (α2-3 linked sialic acid), α-2,6Neu5Ac (α2-6 linked sialic acid), α-2,8Neu5Ac (α2-8 linked sialic acid), sialic acid (α-2,3Neu5Ac, α-2,6Neu5Ac or α-2,8Neu5Ac), Neu5Acα4/9-O-Ac-Neu5Ac, Neu5Acα2-3Galβ1-4Glc/GlcNAc, Neu5Acα2-6Gal/GalNAc, N-linked bi-antennary, N-linked tri/tetra-antennary, branched β1-6GlcNAc, Galα1-3(Fucα1-2)Galβ1-3/4GlcNAc, Galβ1-3(Fucα1-4)GlcNAc, NeuAcα2-3Galβ1-3(Fucα1-4)GlcNAc, Fucα1-2Galβ1-3(Fucα1-4)GlcNAc, Galβ1-4(Fucα1-3)GlcNAc, NeuAcα2-3Galβ1-4(Fucα1-3)GlcNAc, Fucα1-2Galβ1-4(Fucα1-3)GlcNAc, high mannose, sialyl Lewis^{a} (sialyl Le^{a}) antigen, sialyl Lewis^{x} (sialyl Le^{x}) antigen, Lewis^{x} (Le^{x}) antigen, sialyl Tn antigen, sialyl T antigen, Lewis^{y} (Le^{y}) antigen, sulfated core1 glycan, Tn antigen, T antigen, core 2 glycan, Lewis^{a} (Le^{a}) antigen, (GlcNAcβ1-4)ₙ, β-D-GlcNAc, GalNAc, Gal-GlcNAc, GlcNAc, Galα1-3Gal, Galβ1-3GalNAc, α-Gal, α-GalNAc, (GlcNAc)ₙ, branched (LacNAc)ₙ.

Carbohydrate abbreviations as used herein include: "Neu5Ac" for N-acetylneuraminic acid; "Fuc" for fucose, "GalNAc" for N-acetylgalactosamine; "GlcNAc" for *N*-acetylglucosamine; "Gal" for galactose (e.g., Varki A, Cummings RD, Esko JD, Freeze HH, Stanley P, Bertozzi CR, Hart GW, E. ME., Essentials of Glycobiology, 2nd edition, Cold Spring Harbor Laboratory Press, Cold Spring Harbor (NY), 2009).

Furthermore, as used herein the following terms are defined below:
"core fucose" means fucose is linked via an α-glycosidic bond of its C1 atom to the C6 atom of *N*-acetylglucosamine,
"antennary fucose" means fucose is linked via an α-glycosidic bond of its C1 atom to the C3 atom of *N*-acetylglucosamine or fucose is linked via an α-glycosidic bond of its C1 atom to the C2 atom of neighboring fucose,
"Fucα1-6GlcNAc-*N*-Asn containing N-linked oligosaccharides" means oligosaccharides which have fucose linked via a α-glycosidic bond of its C1 atom to the C6 atom of **N-**acetylglucosamine, which is linked to asparagine via *N*-glycosidic bond,
"Fucα1-6/3GlcNAc" means fucose is linked via a α-glycosidic bond of its C1 atom to the C6 (C3) atom of *N*-acetylglucosamine,
"α-L-Fuc" means α-L-fucose,
"Fucα1-2Galβ1-4(Fucα1-3)GlcNAc" means fucose is linked via an α-glycosidic bond of its C1 atom to the C2 atom of galactose, which is linked via an β glycosidic bond of its C1 atom to the C4 atom of *N*-acetylglucosamine; at the same time second fucose is linked via an α-glycosidic bond of its C1 atom to the C3 atom of *N*-acetylglucosamine,
"Fucα1-2Gal" means fucose is linked via a α-glycosidic bond of its C1 atom to the C2 atom of galactose,
"Fucα1-6GlcNAc" means fucose is linked via a α-glycosidic bond of its C1 atom to the C6 atom of *N*-acetylglucosamine,
"Manβ1-4GlcNAcβ1-4GlcNAc" means mannose is linked via a β-glycosidic bond of its C1 atom to the C4 atom of *N*-acetylglucosamine, which is linked via a β-glycosidic bond of its C1 atom to the C4 atom of *N*-acetylglucosamine,
"branched *N*-linked hexa-saccharide" means non-linear glycan composed of several carbohydrates linked to asparagine by *N*-glycosidic bond
"Manα1-3Man" means mannose is linked via a α-glycosidic bond of its C1 atom to the C3 atom of mannose,
"α-D-Man" means α-D-mannose,
"(GlcNAcβ1-4)₂₋₄" means *N*-acetylglucosamine is linked via a β-glycosidic bond of its C1 atom to the C4 atom of *N*-acetylglucosamine repeatedly,
"Galβ1-4GlcNAc" means galactose is linked via a β-glycosidic bond of its C1 atom to the C4 atom of *N*-acetylglucosamine,
"GlcNAcα1-4Galβ1-4GlcNAc" means *N*-acetylglucosamine is linked via a α-glycosidic bond of its C1 atom to the C4 atom of galactose, which is linked via a β-glycosidic bond of its C1 atom to the C4 atom of *N*-acetylglucosamine,
"*N*-acetylglucosamine" means amide between glucosamine and acetic acid,
"(GlcNAcβ1-4)₂₋₅" means *N*-acetylglucosamine is linked via a β-glycosidic bond of its C1 atom to the C4 atom of *N*-acetylglucosamine repeatedly,
"Neu5Ac" (or sialic acid) means *N*-acetylneuraminic acid,
"Galβ1-3GalNAc-serine/threonine" means galactose is linked via a β-glycosidic bond of its C1 atom to the C3 atom of *N*-acetylglucosamine, which is linked to serine/threonine,
"Galα1-3GalNAc" means galactose is linked via a α-glycosidic bond of its C1 atom to the C3 atom of *N*-acetylgalactosamine,
"Galβ1-6Gal" means galactose is linked via a β-glycosidic bond of its C1 atom to the C6 atom of galactose,
"Galβ1-4GlcNAc" means galactose is linked via a β-glycosidic bond of its C1 atom to the C3 atom of *N*-acetylglucosamine,
"Galβ1-3GalNAc" means galactose is linked via a β-glycosidic bond of its C1 atom to the C3 atom of *N*-acetylgalactosamine,
"GalNAcα1-3GalNAc" means *N*-acetylgalactosamine is linked via a α-glycosidic bond of its C1 atom to the C3 atom of *N*-acetylgalactosamine,
"GalNAcα1-3Gal" means *N*-acetylgalactosamine is linked via a α-glycosidic bond of its C1 atom to the C3 atom of galactose,
"GalNAcα/β1-3/4Gal" means *N*-acetylgalactosamine is linked via a α- or β-glycosidic bond of its C1 atom to the C3 or C4 atom of galactose,
"α-GalNAc" means amide between α-galactosamine and acetic acid,
"GalNAcβ1-4Gal" means *N*-acetylgalactosamine is linked via a β-glycosidic bond of its C1 atom to the C4 atom of galactose,
"GalNAcα1-3(Fucα1-2)Gal" means *N*-acetylgalactosamine is linked via a α-glycosidic bond of its C1 atom to the C3 atom of galactose, at the same time fucose is linked via a α-glycosidic bond of its C1 atom to the C2 atom of galactose,
"GalNAcα1-2Gal" means *N*-acetylgalactosamine is linked via a α-glycosidic bond of its C1 atom to the C3 atom of galactose,
"GalNAcα1-3GalNAc" means *N*-acetylgalactosamine is linked via a α-glycosidic bond of its C1 atom to the C3 atom of *N*-acetylgalactosamine,
"GalNAcβ1-3/4Gal" means *N*-acetylgalactosamine is linked via a β-glycosidic bond of its C1 atom to the C3 or C4 atom of galactose,
"GalNAc-Ser/Thr" (or Tn antigen,) means *N-*acetylgalactosamine is linked to serine/threonine via *O*-glycosidic bond,
"Galβ1-3GalNAc-Ser/Thr" (T antigen or Thomsen-Friedenreich antigen) means galactose is linked via a β-glycosidic bond of its C1 atom to the C3 atom of *N*-acetylgalactosamine, which is linked to serine/threonine via *O*-glycosidic bond,
"GalNAcβ1-4GlcNAc" (or LacdiNAc) means *N*-acetylgalactosamine is linked via a β-glycosidic bond of its C1 atom to the C4 atom of *N*-acetylglucosamine,
"α2-3Neu5Ac" (or α2-3-linked sialic acid) means *N*-acetylneuraminic acid is linked via a α-glycosidic bond of its C2 atom to the C3 atom of a neighboring saccharide,
"α2-6Neu5Ac" (or α2-6-linked sialic acid) means *N*-acetylneuraminic acid is linked via a α-glycosidic bond of its C2 atom to the C6 atom of a neighboring saccharide,
"α2-8Neu5Ac" (or α2-8-linked sialic acid) means *N*-acetylneuraminic acid is linked via a α-glycosidic bond of its C2 atom to the C8 atom of a neighboring *N*-acetylneuraminic acid,
"Neu5Acα4/9-O-Ac-Neu5Ac" means *N*-acetylneuraminic acid is linked via a α-glycosidic bond of its C4 atom to the C9 atom of a neighboring *O*-acetyl *N*-acetylneuraminic acid,
"Neu5Acα2-3Galβ1-4Glc/GlcNAc" means *N*-acetylneuraminic acid is linked via a α-glycosidic bond of its C2 atom to the C3 atom of galactose, which is linked via β-glycosidic bond of its C1 atom to the C4 atom of glucose or *N*-acetylglucosamine,
"Neu5Acα2-6Gal/GalNAc" means *N*-acetylneuraminic acid is linked via a α-glycosidic bond of its C2 atom to the C6 atom of galactose or *N*-acetylgalactosamine,
"*N*-linked bi-antennary" means non-linear glycan with two antennas (carbohydrate chains) linked to asparagine by *N*-glycosidic bond,
"*N*-linked tri/tetra-antennary" means non-linear glycan with three/tetra antennas (carbohydrate chains) linked to asparagine by *N*-glycosidic bond,
"branched β1-6GlcNAc" means *N*-acetylglusosamine is linked via a β-glycosidic bond of its C1 atom to the C6 atom of neighboring saccharide,
"Galα1-3(Fucα1-2)Galβ1-3/4GlcNAc" means galactose is linked via a α-glycosidic bond of its C1 atom to the C3 atom of galactose, which is linked via a β-glycosidic bond of its C1 atom to the C3 or C4 atom of *N*-acetylglucosamine; at the same time fucose is linked via a α-glycosidic bond of its C1 atom to the C2 atom of *N*-acetylglucosamine,
"Galβ1-3(Fucα1-4)GlcNAc" means galactose is linked via a β-glycosidic bond of its C1 atom to the C3 atom of *N*-acetylglusosamine; at the same time fucose is linked via α-glycosidic bond of its C1 atom to the C4 atom of *N*-acetylglucosamine,
"NeuAcα2-3Galβ1-3(Fucα1-4)GlcNAc" means *N*-acetylneuraminic acid is linked via a α-glycosidic bond of its C2 atom to the C3 atom of galactose, which is linked via β-glycosidic bond of its C1 atom to the C3 atom *N*-acetylglucosamine; at the same time fucose is linked via a α-glycosidic bond of its C1 atom to the C4 atom of *N*-acetylglucosamine,
"Fucα1-2Galβ1-3(Fucα1-4)GlcNAc" means fucose is linked via a α-glycosidic bond of its C1 atom to the C2 atom of galactose, which is linked via β-glycosidic bond of its C1 atom to the C3 atom *N*-acetylglucosamine; at the same time second fucose is linked via α-glycosidic bond of its C1 atom to the C4 atom of *N*-acetylglucosamine,
"Galβ1-4(Fucα1-3)GlcNAc" means galactose is linked via β-glycosidic bond of its C1 atom to the C4 atom of *N*-acetylglucosamine; at the same time fucose is linked via α-glycosidic bond of its C1 atom to the C3 atom of *N*-acetylglucosamine,
"NeuAcα2-3Galβ1-4(Fucα1-3)GlcNAc" means *N*-acetylneuraminic acid is linked via α-glycosidic bond of its C2 atom to the C3 atom of galactose, which is linked via β-glycosidic bond of its C1 atom to the C4 atom *N*-acetylglucosamine; at the same time fucose is linked via α-glycosidic bond of its C1 atom to the C3 atom of *N*-acetylglucosamine,
"Fucα1-2Galβ1-4(Fucα1-3)GlcNAc" means fucose is linked via α-glycosidic bond of its C1 atom to the C2 atom of galactose, which is linked via β-glycosidic bond of its C1 atom to the C4 atom *N*-acetylglucosamine; at the same time second fucose is linked via α-glycosidic bond of its C1 atom to the C3 atom of *N*-acetylglucosamine,
"high mannose" means glycan containing more than three mannose units,
"sialyl Lewis^{a}" (sialyl Le^{a}) antigen is Neu5Acα,2-3/6Galβ1-3(Fucα1-4)GlcNAc meaning *N-*acetylneuraminic acid is linked via α-glycosidic bond of its C2 atom to the C3 or C6 atom of galactose, which is linked via β-glycosidic bond of its C1 atom to the C3 atom *N-*acetylglucosamine; at the same time fucose is linked via α-glycosidic bond of its C1 atom to the C4 atom of *N*-acetylglucosamine,
"sialyl Lewis^{x} " (sialyl Le^{x}) antigen is Neu5Acα2-3/6Galβ1-4(Fucα1-3)GlcNAc meaning *N-*acetylneuraminic acid is linked via α-glycosidic bond of its C2 atom to the C3 or C6 atom of galactose, which is linked via β-glycosidic bond of its C1 atom to the C4 atom *N-*acetylglucosamine; at the same time fucose is linked via α-glycosidic bond of its C1 atom to the C3 atom of *N*-acetylglucosamine,
"Lewis^{x} " (Le^{x}) antigen is "Galβ1-4(Fucα1-3)GlcNAc" meaning galactose is linked via β-glycosidic bond of its C1 atom to the C4 atom of *N*-acetylglucosamine; at the same time fucose is linked via α-glycosidic bond of its C1 atom to the C3 atom of *N*-acetylglucosamine,
"sialyl Tn antigen" is "Neu5Acα2-3/6GalNAc-Ser/Thr" meaning *N*-acetylneuraminic acid is linked via α-glycosidic bond of its C2 atom to the C3 or C6 atom of *N*-acetylgalactosamine, which is linked to serine/threonine via *O*-glycosidic bond,
"sialyl T antigen" is "Neu5Acα2-3/6Galβ1-3GalNAc-Ser/Thr" meaning *N*-acetylneuraminic acid is linked via α-glycosidic bond of its C2 atom to the C3 or C6 atom of galactose, which is linked via β-glycosidic bond of its C1 atom to the C3 atom of *N*-acetylgalactosamine, which is linked to serine/threonine via *O*-glycosidic bond,
"Lewis^{y} " (Le^{y}) antigen is "Fucα1-2Galβ1-4(Fucα1-3)GlcNAc" meaning fucose is linked via α-glycosidic bond of its C1 atom to the C2 atom of galactose, which is linked via β-glycosidic bond of its C1 atom to the C4 atom of *N*-acetylglucosamine; at the same time second fucose is linked via α-glycosidic bond of its C1 atom to the C3 atom of *N-*acetylglucosamine,
"sulfated core1 glycan" is a glycan based on suflated extended form of T antigen,
"core 2 glycan" is a glycan based on an extended form of Galβ1-3(GlcNAcβ1-6)GalNAc-Ser/Thr meaning an extended form of glycan having galactose linked via β-glycosidic bond of its C1 atom to the C3 atom of *N*-acetylgalactosamine, at the same time *N*-acetylglucosamine is linked via β-glycosidic bond of its C1 atom to the C6 atom of *N*-acetylgalactosamine, which is linked to serine/threonine
"Lewis^{a} " (Le^{a}) antigen is Galβ1-3(Fucα1-4)GlcNAc meaning galactose is linked via β-glycosidic bond of its C1 atom to the C3 atom *N*-acetylglucosamine; at the same time fucose is linked via α-glycosidic bond of its C1 atom to the C4 atom of *N*-acetylglucosamine,
"(GlcNAcβ1-4)ₙ" means *N*-acetylglucosamine is linked via β-glycosidic bond of its C1 atom to the C4 atom of *N*-acetylglucosamine repeatedly,
"β-D-GlcNAc" means amide between β-D-glucosamine and acetic acid,
"GalNAc" means amide between galactosamine and acetic acid i.e. *N*-acetylgalactosamine,
"Gal-GlcNAc" means galactose is linked to *N*-acetylglucosamine via non-specified linkage,
"GlcNAc" means amide between glucosamine and acetic acid i.e. *N*-acetylglucosamine.
"Galα1-3Gal" means galactose is linked via α-glycosidic bond of its C1 atom to the C3 atom of galactose,
"Galβ1-3GalNAc" means galactose is linked via β-glycosidic bond of its C1 atom to the C3 atom of *N*-acetylgalactosamine,
"α-Gal" means α-galactose,
"α-GalNAc" means amide between α -D-galactosamine and acetic acid,
"(GlcNAc)ₙ" means *N*-acetylglucosamine is linked to *N*-acetylglucosamine via non-specified linkage,
"branched (LacNAc)ₙ" is branched and repeated form of Galβ1,4-GlcNAc meaning a branched and repeated form of galactose linked via β-glycosidic bond of its C1 atom to the C4 atom of N-acetylglucosamine.

The "signal" as used herein relates to the information obtained by determining a glycan structure (A) (suspected to be) on a protein of interest. This information typically is signal intensity, e.g., of an absorption at a particular wave length or fluorescence emission at a particular wave length. The signal intensity preferably is dependent on the amount of glycan structure (A) present on the protein of interest. The signal may thus also be seen as providing information on the glycoprofile of the protein of interest.

The neoglycoprotein described herein acts as a standard in methods and uses of the invention. These relate to determining a glycan structure (A) providing a signal (1) or a signal (2). "Determining a glycan structure (A)" as used herein relates to any method that is suitable for assaying whether a particular glycan structure (A) is present on a protein of interest or not, preferably suitable for determining the amount of glycan structure (A) (in a sample). In other words, determining a glycan structure (A) may also be seen as a method of glycoprofiling the protein interest suspected to have the glycan structure (A) on it. Thus, this method provides the signal that is relativized (signal (1)) or is the basis for the relativizing (signal (2)). Suitable methods include, but are not limited to, enzyme-linked immunosorbent assay (ELISA), enzyme-linked lectin assay (ELLA), magnetic ELLA (MELLA), preferably ELLA or MELLA. Accordingly, signal (1) and signal (2) may be obtained by enzyme-linked immunosorbent assay (ELISA), enzyme-linked lectin assay (ELLA), magnetic ELLA (MELLA), preferably ELLA or MELLA. Preferably, signal (1) and signal (2) are obtained at the same conditions, including the same reagents at the same concentrations. The present invention is however not limited to these methods but may include any other assay method involving glycan analysis.

ELISA can be seen as the starting point to a variety of similar or ELISA-like assays. ELISA is based on the specific interaction of an immunoglobulin with a molecule of interest such as the glycan structure (A) suspected to be on the protein of interest, wherein the specific interaction allows the generation of a signal only in presence of the molecule of interest and the signal (strength) corresponds to the concentration of the molecule of interest in the analyzed sample. Assays like ELISA, ELLA and MELLA are known to a person skilled in the art. Various ELISA variations such as sandwich ELISA, competitive ELISA and reverse ELISA are further known in the art. In the context of the invention, the primary immunoglobulin, i.e. the immunoglobulin binding to the molecule of interest, binds to the glycan structure (A) suspected to be on the protein of interest. Thus, the primary immunoglobulin, preferably an antibody or a fragment thereof, specifically binds to a glycan structure (A) of interest. In ELLA, the primary immunoglobulin is replaced by a lectin, which binds specifically to a glycan structure (A). Magnetic ELLA (MELLA) is a further development of ELLA described in WO 2019/185515 A1. The readout of these assays, e.g. the signal intensity, can be seen as the signal (1) or signal (2) in the context of the invention. Typically, the readout is the result of an enzymatic reaction of an enzyme coupled to a second immunoglobulin that binds to the primary immunoglobulin, to the protein of interest (to the protein backbone of the glycoprotein of interest) or the lectin. Alternatively, the enzyme can (directly) be coupled to the lectin. Frequently used readouts include, but are not limited to, OPD (o-phenylenediamine dihydrochloride) turning amber to detect HRP (Horseradish Peroxidase), which is often used to as a conjugated protein; TMB (3,3',5,5'-tetramethylbenzidine) turning blue when detecting HRP and turns yellow after the addition of sulfuric or phosphoric acid; ABTS (2,2'-Azinobis [3-ethylbenzothiazoline-6-sulfonic acid]-diammonium salt) turning green when detecting HRP; or PNPP (p-Nitrophenyl Phosphate, Disodium Salt) turning yellow when detecting alkaline phosphatase. In addition to these colorimetric readouts, also radiolabel, fluorescent label, electrochemical label, chemiluminescent label, colorimetric approach or even a label-free format (e.g. SPR) can be used. Another example is 10-acetyl-3,7-dihydroxyphenoxazine (Amplex^{®} Red reagent) to detect hydrogen peroxide (H2O2) or peroxidase activity. In the presence of peroxidase, the Amplex^{®} Red reagent reacts with H₂O₂ in a 1:1 stoichiometry to produce the red-fluorescent oxidation product, resorufin. Resorufin has excitation and emission maxima of approximately 571 nm and 585 nm.

The term "lectin" when used herein refers to a carbohydrate-binding protein. A lectin typically is highly specific for a carbohydrate moiety or carbohydrate moieties (e.g., it reacts specifically with terminal glycosidic residues of other molecules such as a glycan/s of a glycoprotein (e.g., branching sugar molecules of glycoproteins, e.g., such as target polypeptides within the meaning of the present invention and biomarkers as described in **Table 1** herein). Lectins are commonly known in the art. A skilled person is readily available to determine which lectin may be used for binding a carbohydrate moiety or carbohydrate moieties of interest, e.g. a carbohydrate moiety or carbohydrate moieties of a glycan attached to a protein. Preferred lectins applied in the context of the present invention are described herein. Also included by the term "lectin" are Siglecs (sialic acid-binding immunoglobulin-like lectins), Galectins (lectins that bind specifically to β-galactoside containing glycans) and Selectins (bind to the sialyl Lewis X (SLe^{x}) determinant NeuAcα2-3Galβ1-4(Fucα1-3)GlcNAc and related sialylated, fucosylated glycans). Notably, the term "lectin" when used herein also refers to glycan-binding antibodies. Accordingly, the term "lectin" when used herein may also encompass lectins, Siglecs, Galectins, Selectins, etc. as well as glycan-binding antibodies. Lectins may also include DNA/RNA aptamers recognizing glycans.

Lectins can be obtained from seeds of leguminous plants, but also from other plant and animal sources. Lectins can contain binding sites for specific mono- and oligosaccharides (e.g., glycans of glycoproteins). They can agglutinate cells by binding to specific sugar residues in membrane glycoproteins. Preferably, lectins of the present invention are selected from the group consisting of: *Maackia amurensis* lectin II (MAA II); Concanavalin A (Con A); *Aleuria aurantia* lectin (AAL); *Sambucus nigra* (SNA-I) lectin; *Wisteria floribunda* lectin (WFL) as defined herein.

Further preferred lectins of the present invention are shown in **Table 1** below.

Particularly preferred lectins of the present invention are lectins with the following UniProtKB Accession Numbers: P0DKL3, P02866, P18891, 004366, A0A218PFP3, Q945S3, Q00022, Q6YNX3, Q71QF2, P02872, P18670, Q2UNX8, Q8L5H4, A0A089ZWN7, P05045, P19588, P83410, P17931, P56470, P24146, Q41263, Q39990, Q2F1K8, G9M5T0, B3XYC5, P02870, P19664, P0DKL3, P49300, A9XX86, Q40423, P16300, P05088, P05087, Q9AVB0, P02867, 024313, Q9SM56, P06750, B9SPG3, Q9BZZ2, P20916, Q9NYZ4, Q96RL6, P05046, P93535, P02876, P10968, P10969, P22972 or P56625 as well as corresponding mature forms thereof.

Exemplary lectins of the present invention further include:
*Maackia amurensis* lectin II (MAA II) is the hemagglutinin isolectin from *Maackia* seeds. Sialic acid-binding lectin recognizing oligosaccharides containing terminal sialic acid linked via α2-3 bond to neighboring galactose residues. Binds the trisaccharide sequence Neu5Acα2-3-Gal-β-1-4-GlcNAc. Preferably, MAA II has a SEQ ID NO: 2 (or its mature form).
Concanavalin A (Con A) a D-mannose specific lectin originally extracted from the jack-bean, *Canavalia ensiformis.* Preferably, Con A has a SEQ ID NO: 3 or SEQ ID NO: 4 (Con A, mature form).
*Aleuria aurantia* lectin (AAL) is a fucose-specific lectin extracted from *Aleuria aurantia* (Orange peel mushroom). Preferably, AAL has a SEQ ID NO: 5 (or its mature form). The isolation of AAL is, for example, described in (Debray et al., Kochibe et al.).
*Sambucus nigra* (SNA-I) lectin is a Neu5Acα2-6)Gal/GalNAc specific agglutinin extracted from *Sambucus nigra* (European elder). Preferably, SNA-I has a SEQ ID NO: 6 (or its mature form).
*Wisteria floribunda* lectin (WFL) is an agglutinin extracted from *Wisteria floribunda* (Japanese wisteria). Preferably, WFL has a SEQ ID NO: 7 (or its mature form).

Furthermore, suitable lectins within the meaning of the present invention may explicitly include post-translationally processed- and mature forms of the lectins as disclosed herein.

The term "antibody" also includes, but is not limited to, but encompasses monoclonal, monospecific, poly- or multi-specific antibodies such as bispecific antibodies, humanized, camelized, human, single-chain, chimeric, synthetic, recombinant, hybrid, mutated, grafted, and in vitro generated antibodies, with chimeric or humanized antibodies being preferred. The term "humanized antibody" is commonly defined for an antibody in which the specificity encoding CDRs of HC and LC have been transferred to an appropriate human variable framework ("CDR grafting"). The term "antibody" also includes scFvs, single chain antibodies, diabodies or tetrabodies, domain antibodies (dAbs) and nanobodies. In terms of the present invention, the term "antibody" shall also comprise bi-, tri- or multimeric or bi-, tri- or multifunctional antibodies having several antigen binding sites. Said term also includes antigen binding portion(s). Also included by the term "antibody" may be FN3 scaffold, adnectin, affibody, anticalin, avimer, a bicyclic peptide, DARPin, a Kunitz domain, an Obody or an aptamer, such as a DNA, RNA or peptide aptamer.

Preferred antibodies of the present invention include, but are not limited to, an anti-PSA, anti-AFP, anti-MUC16, anti-WFDC2, anti-MUC1, anti-ERBB2, anti-CEACAM5, anti-FUT3 or anti-TG antibodies etc. Further preferred antibodies relating to the present invention are shown in **Table 1** below.

Furthermore, the term "antibody" as employed in the invention also relates to derivatives of the antibodies (including fragments) described herein. A "derivative" of an antibody comprises an amino acid sequence which has been altered by the introduction of amino acid residue substitutions, deletions or additions. Additionally, a derivative encompasses antibodies which have been modified by a covalent attachment of a molecule of any type to the antibody or protein. Examples of such molecules include sugars, PEG, hydroxyl-, ethoxy-, carboxy- or amine-groups but are not limited to these. In effect the covalent modifications of the antibodies lead to the glycosylation, pegylation, acetylation, phosphorylation, amidation, without being limited to these.

The protein of interest is not limited to a particular protein. In contrast and due to the versatility of the neoglycoprotein, the methods and uses of the invention can be applied to assay whether a particular glycan structure (A) is present on the protein of interest. However, the protein of interest preferably is a glycoprotein or, to put it differently, a protein which is suspected to have a glycan structure (A). Since the presence or absence of a glycan structure (A) on the protein of interest may be important for diagnosis or prognosis of a disease, the protein of interest preferably is a protein, whose glycoprofile is relevant for a disease. The term "glycoprotein" (or "glycosylated protein") as used herein means a protein containing one or more *N*-, *O-, S*- or *C*- covalently linked carbohydrates of various types, e.g., ranging from monosaccharides to branched polysaccharides (including their modifications such as sulfo- or phospho- group attachment). *N*-linked glycans are carbohydrates bound to -NH₂ group of asparagine. O-linked glycans are carbohydrates bound to -OH group of serine, threonine, or hydroxylated amino acids. *S*-linked glycans are carbohydrates bound to -SH group of cysteine. C-linked glycans are carbohydrates bound to tryptophan *via* C-C bond.

The term "carbohydrates" means compounds (e.g., such as aldoses and ketoses) having the stoichiometric formula Cₙ(H₂O)ₙ. The generic term "carbohydrate" includes monosaccharides, oligosaccharides and polysaccharides as well as substances derived from monosaccharides by reduction of the carbonyl group (alditols), by oxidation of one or more terminal groups to carboxylic acids, or by replacement of one or more hydroxy group(s) by a hydrogen atom, an amino group, thiol group or similar groups. It also includes derivatives of these compounds.

As already described herein, the presence of a particular glycan structure (A) on the protein of interest may be relevant for the diagnosis of a particular disease such as a cancer, an autoimmune disease or an inflammatory disease. Some combinations of proteins (of interest, i.e. biomarker proteins) and glycan structures (A) are known to be indicative for diseases. Specific combinations of proteins of interest and glycans indicative for diseases are exemplified in **Table 1** as well as antibodies or lectins binding to the particular glycan structures. Thus, the method and uses of the present invention can be used in diagnosing of diseases such as cancer, autoimmune disease or inflammatory disease. Accordingly, the presence of said glycan structure (A) may be indicative of a disease such as cancer, autoimmune disease or inflammatory disease.

In this context, the protein of interest preferably is a cancer biomarker protein, an autoimmune disease biomarker protein or an inflammatory disease biomarker protein.

As used herein, an "autoimmune disease" refers a group of diseases characterized by disease associated with the production of antibodies directed against one's own tissues. Non-limiting examples of an autoimmune disease include, but are not limited to, Hashimoto's disease, primary biliary cirrhosis, systemic lupus erythematosus, rheumatic fever, rheumatoid arthritis, autoimmune hemolytic anemia, idiopathic thrombocytopenic purpura, and postviral encephalomyelitis, Addison's disease, autoimmune enteropathy, primary biliary cirrhosis, Goodpasture's syndrome, Hashimoto's thyroiditis, myasthenia gravis, myxoedema, pemphigoid, rheumatoid arthritis, Sjogren's syndrome, symphathetic ophthalmitis, both forms of lupus erythematosus, thyrotoxicosis, ulcerative colitis, multiple sclerosis, celiac disease, diabetes mellitus type 1, Graves' disease, inflammatory bowel disease and psoriasis.

As used herein, an "inflammatory disease" refers a group of diseases characterized by impairment and/or abnormal functioning of inflammatory mechanisms of the body. Non-limiting examples of an inflammatory disease include, but are not limited to, necrotizing enterocolitis, gastroenteritis, pelvic inflammatory disease (PID), empyema, pleurisy, pyelitis, pharyngitis, angina, arthritis, acne, urinary tract infections, Acne vulgaris, Asthma, Celiac disease, Chronic prostatitis, Colitis, Diverticulitis, Glomerulonephritis, Hidradenitis suppurativa, Hypersensitivities, Inflammatory bowel diseases, Interstitial cystitis, Mast Cell Activation Syndrome, Mastocytosis, Otitis, Pelvic inflammatory disease, Reperfusion injury, Rheumatic fever, Rheumatoid arthritis, Rhinitis, Sarcoidosis, Transplant rejection, Vasculitis.

As used herein, "cancer" refers a group of diseases characterized by the uncontrolled growth of abnormal cells in the body. Unregulated cell division may result in the formation of malignant tumours or cells that invade neighbouring tissues and may metastasize to distant parts of the body through the lymphatic system or bloodstream. Non-limiting examples of cancers include squamous cell carcinoma, small-cell lung cancer, non- small cell lung cancer, squamous non-small cell lung cancer (NSCLC), non NSCLC, glioma, gastrointestinal cancer, renal cancer

(e.g. clear cell carcinoma), ovarian cancer, liver cancer, colorectal cancer, endometrial cancer, kidney cancer (e.g., renal cell carcinoma (RCC)), prostate cancer (e.g. hormone refractory prostate adenocarcinoma), thyroid cancer, neuroblastoma, pancreatic cancer, glioblastoma (glioblastoma multiforme), cervical cancer, stomach cancer, bladder cancer, hepatoma, breast cancer, colon carcinoma, and head and neck cancer (or carcinoma), gastric cancer, germ cell tumour, pediatric sarcoma, sinonasal natural killer, melanoma (e.g., metastatic malignant melanoma, such as cutaneous or intraocular malignant melanoma), bone cancer, skin cancer, uterine cancer, cancer of the anal region, testicular cancer, carcinoma of the fallopian tubes, carcinoma of the endometrium, carcinoma of the cervix, carcinoma of the vagina, carcinoma of the vulva, cancer of the oesophagus, cancer of the small intestine, cancer of the endocrine system, cancer of the parathyroid gland, cancer of the adrenal gland, sarcoma of soft tissue, cancer of the urethra, cancer of the penis, solid tumours of childhood, cancer of the ureter, carcinoma of the renal pelvis, neoplasm of the central nervous system (CNS), primary CNS lymphoma, tumour angiogenesis, spinal axis tumour, brain stem glioma, pituitary adenoma, Kaposi's sarcoma, epidermoid cancer, squamous cell cancer, T-cell lymphoma, environmentally-induced cancers including those induced by asbestos, virus-related cancers (e.g., human papilloma virus (HPV)- related tumour), and hematologic malignancies derived from either of the two major blood cell lineages, i.e., the myeloid cell line (which produces granulocytes, erythrocytes, thrombocytes, macrophages and mast cells) or lymphoid cell line (which produces B, T, NK and plasma cells), such as all types of leukaemia, lymphomas, and myelomas, e.g., acute, chronic, lymphocytic and/or myelogenous leukaemia, such as acute leukaemia (ALL), acute myelogenous leukaemia (AML), chronic lymphocytic leukaemia (CLL), and chronic myelogenous leukaemia (CML), undifferentiated AML (MO), myeloblastic leukaemia (MI), myeloblastic leukaemia (M2; with cell maturation), promyelocytic leukaemia (M3 or M3 variant [M3V]), myelomonocytic leukaemia (M4 or M4 variant with eosinophilia [M4E]), monocytic leukaemia (M5), erythroleukaemia (M6), megakaryoblastic leukaemia (M7), isolated granulocytic sarcoma, and chloroma; lymphomas, such as Hodgkin' s lymphoma (HL), non-Hodgkin' s lymphoma (NHL), B-cell lymphomas, T-cell lymphomas, lymphoplasmacytoid lymphoma, monocytoid B-cell lymphoma, mucosa-associated lymphoid tissue (MALT) lymphoma, anaplastic (e.g., Ki 1+) large-cell lymphoma, adult T-cell lymphoma/leukaemia, mantle cell lymphoma, angio immunoblastic T-cell lymphoma, angiocentric lymphoma, intestinal T-cell lymphoma, primary mediastinal B-cell lymphoma, precursor T-lymphoblastic lymphoma, T-lymphoblastic; and lymphoma/leukaemia (T-Lbly/T-ALL), peripheral T- cell lymphoma, lymphoblastic lymphoma, post-transplantation, lymphoproliferative disorder, true histiocytic lymphoma, primary central nervous system lymphoma, primary effusion lymphoma, lymphoblastic lymphoma (LBL), hematopoietic tumours of lymphoid lineage, acute lymphoblastic leukaemia, diffuse large B-cell lymphoma, Burkitt's lymphoma, follicular lymphoma, diffuse histiocytic lymphoma (DHL), immunoblastic large cell lymphoma, precursor B-lymphoblastic lymphoma, cutaneous T-cell lymphoma (CTLC) (also called mycosis fungoides or Sezary syndrome), and lymphoplasmacytoid lymphoma (LPL) with Waldenstrom's macroglobulinemia; myelomas, such as IgG myeloma, light chain myeloma, non-secretory myeloma, smouldering myeloma (also called indolent myeloma), solitary, plasmocytoma, and multiple myelomas, chronic lymphocytic leukaemia (CLL), hairy cell lymphoma; hematopoietic tumours of myeloid lineage, tumours of mesenchymal origin, including fibrosarcoma and rhabdomyosarcoma; seminoma, teratocarcinoma, tumours of the central and peripheral nervous, including astrocytoma, schwannomas; tumours of mesenchymal origin, including fibrosarcoma, rhabdomyosarcoma, and osteosarcoma; and other tumours, including melanoma, xeroderma pigmentosum, keratoacanthoma, seminoma, thyroid follicular cancer and teratocarcinoma, hematopoietic tumours of lymphoid lineage, for example T-cell and B-cell tumours, including but not limited to T-cell disorders such as T-prolymphocytic leukaemia (T-PLL), including of the small cell and cerebriform cell type; large granular lymphocyte leukaemia (LGL) preferably of the T-cell type; a/d T-NHL hepatosplenic lymphoma; peripheral/post-thymic T cell lymphoma (pleomorphic and immunoblastic subtypes); angiocentric (nasal) T-cell lymphoma; cancer of the head or neck, renal cancer, rectal cancer, cancer of the thyroid gland; acute myeloid lymphoma, as well as any combinations of said cancers. Preferred cancers are also shown in **Table 1.**

The cancer may also be an ovarian cancer, breast cancer, colorectal cancer, pancreatic cancer, prostate cancer, thyroid cancer, liver cancer, lung cancer, stomach cancer, testicular cancer or bladder cancer. Accordingly, the biomarker protein (protein of interest) may be an ovarian cancer biomarker protein, breast cancer biomarker protein, colorectal cancer biomarker protein, pancreatic cancer biomarker protein, prostate cancer biomarker protein, thyroid cancer biomarker protein, liver cancer biomarker protein, lung cancer biomarker protein, stomach cancer biomarker protein, testicular cancer biomarker protein or bladder cancer biomarker protein.

Exemplary cancers, cancer biomarkers with aberrant glycosylation, lectins, antibodies and corresponding glycan modifications within the meaning of the present invention are also shown in **Table 1** below. Lectin abbreviations used in **Table 1:** Cancers, corresponding cancer biomarkers with aberrant glycosylation, lectins and antibodies.: AAA - Anguilla anguilla agglutinin (UniProtKB Accession Number: Q7SIC1), AAL *- Aleuria aurantia* lectin, ABA - *Agaricus bisporus* agglutinin, ACA *- Amaranthus caudatus* agglutinin, AHA *- Arachis hypogaea* agglutinin = peanut agglutinin (PNA), AIA *- Artocarpus integrifolia* agglutinin = Jacalin, AlloA *- Allomyrina dichotoma* agglutinin, AOL *- Aspergillus oryzae* lectin, BanLec - *Musa paradisiaca* lectin, BS-I *- Bandeiraea simplicifolia* lectin = *Griffonia (Bandeiraea) simplicifolia* lectin I, Con A - Concanavalin A, DBA - *Dolichos biflorus* agglutinin, DSA - *Datura stramonium* agglutinin (Jacalin), ECL - *Erythrina cristagalli* lectin, GNA - *Galanthus nivalis* agglutinin, GSA I (GSL I) *- Griffonia (Bandeiraea) simplicifolia* lectin I, GSL II - *Griffonia* (*Bandeiraea*) *simplicifolia* lectin II, HHL *- Hippeastrum* hybrid (*Amaryllis*) lectin, HPA - *Helix pomatia* agglutinin, LBA - *Phaseolus lunatus* (lima bean, LBA), LEL - *Lycopersicon esculentum* (tomato) lectin, LCA *- Lens culinaris* agglutinin, LTA - *Lotus tetragonolobus* lectin, MAA I *- Maackia amurensis* agglutinin I, MAA II *- Maackia amurensis* agglutinin II, MGBL 1 - macrophage galactose binding lectin 1, MGBL 2 (macrophage galactose binding lectin 2, NPA *- Narcissus pseudonarcissus* (Daffodil) lectin, PHA E *- Phaseolus vulgaris* agglutinin E, PHA L *- Phaseolus vulgaris* agglutinin L, PhoSL - *Pholiota squarrosa* lectin, PNA - Peanut agglutinin, PSL - *Pisum sativum* lectin, PTA I *- Psophocarpus tetragonolobus* lectin I, PTA II *- Psophocarpus tetragonolobus* II, PWM - *Phytolacca americana,* RCA I *- Ricinus communis* agglutinin I, RCA II *- Ricinus communis* agglutinin II, SBA - Soybean agglutinin (*Glycine max* agglutinin), SCA - *Sambucus canadensis* agglutinin = *Sambucus nigra* agglutinin (SNA), SJA - *Sophora japonica* agglutinin II, SNA - *Sambucus nigra* agglutinin, SSA - *Sambucus sieboldiana* agglutinin, SSL - *Salvia sclarea* lectin, STL - *Solanum tuberosum* lectin, TJA-I - *Trichosanthes japonica* agglutinin I, TJA-II - *Trichosanthes japonica* agglutinin (Yamashita et al.), TVA *- Triticum vulgaris* agglutinin = WGA - wheat germ agglutinin, UEA - *Ulex europaeus* agglutinin, VVA *- Vicia villosa* lectin, WFA - *Wisteria floribunda* lectin, WGA - wheat germ agglutinin = TVA - *Triticum vulgaris* agglutinin. The symbol "↑", an *upward pointing arrow* means increase in concentration of a corresponding glycan/s or a complex/s (e.g., dimer, trimer etc). The symbol "↓", a *downward pointing arrow* means increase in concentration of a corresponding glycan/s or a complex/s (e.g., dimer, trimer etc).

**Table 1: Cancers, corresponding cancer biomarkers with aberrant glycosylation, lectins and antibodies. The combinations of this table are merely examples for different cancer types. The present invention is not limited to these exemplary combinations.**

| **Cancer** | **Biomarker** | **Glycan modification** | **Lectins/antibodie s applied** | **Refs .** | **(Other) applicable lectins/Ab s** |
|---|---|---|---|---|---|
| ***Prostate*** | Prostate specific antigen (PSA) | ↑ α2-3Neu5Ac | MAA | [1-5] | anti-α2-3-linked sialic acid antibody (i.e. i.e. HYB4), Siglec 1, Siglec 4 or Siglec 8 |
| | fPSA | ↑ α2-3Neu5Ac | SNA* (determination of non-eluted PSA | [6, 7] | anti-α2,3-linked sialic acid |
| | | | from SNA affinity column) | | antibody (i.e. HYB4i.e. i.e. HYB4), MAA, Siglec 1, Siglec 4 or Siglec 8 |
| | fPSA | ↑ α2-3 Neu5Ac | anti-α2-3-linked sialic acid antibody (i.e. HYB4) | [8] | MAA, Siglec 1, Siglec 4 or Siglec 8 |
| | PSA [1], tPSA/fPSA [9] | ↓ bi-antennary glycans | Con A | [1, 9] | |
| | PSA [1], tPSA/fPSA [9] | ↓ high mannose glycans | Con A | [1, 9] | GNA, NPA |
| | PSA | ↓ α2-6Neu5Ac | SNA | [1] | TJA-I, SCA |
| | PSA | ↓ α2-6Neu5Ac | TJA-I | [2] | SNA, SCA |
| | PSA | ↑ tri-, tetra-antennary glycans | DSA (Jacalin) | [2] | PHA-L, PHA-E |
| | PSA | ↑ α1-2fucose, GalNAc | TJA-II | [2] | AAL, UEA-I, LCA, PSL, AAA, LTA, HPA, LBA, WFA, VVA |
| | PSA [2], fPSA/tPSA [10] | ↑ α1-2fucose | UEA-I | [2] [10] | TJA II, AAL, LCA, PSL, AAA, LTA |
| | PSA [2], tPSA [11, 12] | ↑ LacdiNAc, GalNAc | WFA | [2, 11, 12] | DBA, SBA, HPA, LBA, VVA |
| | tPSA | ↑ α1-3/6fucose | AAL | [13] | TJA II, UEA-I, LCA, PSL, AAA, LTA, AOL, PhoSL |
| | PSA in urine | ↓ α1-3/6 fucose | AAL | [14] | TJA II, UEA-I, LCA, PSL, AAA, LTA. AOL, PhoSL |
| | PSA in urine | ↓ core fucose (α1-6fucose) | PhoSL | [14] | AOL |
| | fPSA | ↓ core fucose (α1-6fucose) | PhoSL | [6] | AOL |
| | Tissue inhibitor of metallopeptidase 1 (TIMP1) | ↑ α1-3/6fucose | AAL | [13] | AOL, PhoSL, TJA II, UEA-I, LCA, PSL, AAA, LTA |
| | β-haptoglobin | ↑ core fucose (α1-6fucose) | No lectin used, but MS | [15] | PhoSL, AOL |
| | β-haptoglobin | ↑ core/antennar y fucose | AAL | [16, 17] | AOL, PhoSL, TJA II, UEA-I, LCA, PSL, AAA, LTA |
| | β-haptoglobin | ↑ α2-6Neu5Ac | SNA | [16, 17] | TJA-I, SCA |
| | β-haptoglobin | ↑ tri-,tetra-antennary glycans | PHA-L | [16, 17] | PHA-E, DSA (Jacalin) |
| | β-haptoglobin | ↑ sialyl Lewis^{a} glycan | Antibody against sialyl Lewis^{a} glycan | [16] | SNA, TJA-I, MAA, anti-α2-3-linked sialic acid I antibody (i.e. HYB4i.e. i.e. HYB4), Siglec 1, Siglec 4 or Siglec 8 |
| | β-haptoglobin | ↑ sialyl Lewis^{x} glycan | Antibody against sialyl Lewis^{x} glycan | [17] | SNA, TJA-I, MAA, anti-α2-3-linked sialic acid antibody (i.e. HYB4i.e. i.e. HYB4), Siglec 1, Siglec 4 or Siglec 8 |
| | β-haptoglobin | ↑ antennary fucose | No lectin used, but MS | [18] | TJA II, AAL, UEA-I, LCA, PSL, AAA, LTA |
| | β-haptoglobin | ↑ tri-,tetra-antennary glycans | No lectin used, but MS | [18] | PHA-L, PHA-E, DSA |
| | β-haptoglobin | ↑ sialyl Lewis^{a} and sialyl Lewis^{x} glycans | No lectin used, but MS | [18] | Antibodies against sialyl Lewis^{a} and Lewis^{x} glycans, SNA, TJA-I, MAA, anti-α2-3-linked sialic acid antibody (i.e. HYB4i.e. i.e. HYB4), Siglec 1, Siglec 4 or Siglec 8 |
| | β-haptoglobin | ↑ antennary fucose | AAL | [19] | TJA II, UEA-I, LCA, PSL, AAA, LTA |
| | | | | | |
| **Ovarian** | α₁-acid glycoprotein | ↑ tri-,tetra-antennary glycans | Capillary electrophoresis (CE) | [20] | PHA-L, PHA-E, DSA |
| | α₁-acid glycoprotein | ↑ core fucose | CE | [20] | PhoSL, AOL |
| | α₁-acid glycoprotein | ↑ α2-6Neu5Ac | 2D PAGE and LC | [21] | TJA-I, SNA |
| | α₁-acid glycoprotein | ↑ sialyl Le^{x} | 2D PAGE and LC | [21] | Antibody against sLe^{x}, SNA, TJA-I, MAA, anti-α2-3-linked sialic acid antibody (i.e. HYB4i.e. i.e. HYB4), Siglec 1, Siglec 4 or Siglec 8 |
| | α₁-acid glycoprotein | ↓ α2-3Neu5Ac | 2D PAGE and LC | [21] | MAA, anti-α2-3-linked sialic acid antibody (i.e. HYB4i.e. i.e. HYB4), Siglec 1, Siglec 4 or Siglec 8 |
| | C1 esterase inhibitor | ↑ Le^{x} | CE | [20] | Antibody against Le^{x}, LTA |
| | C1 esterase inhibitor | ↑ tri-antennary glycans | CE | [20] | DBA, PHA-E, PHA-L |
| | 2-HS glycoprotein | ↑ tri-,tetra-antennary glycans | CE | [20] | DBA, PHA-E, PHA-L |
| | β-haptoglobin | ↑ tri-,tetra-antennary glycans | CE | [20] | DBA, PHA-E, PHA-L |
| | β-haptoglobin | ↑ Le^{x} | CE | [20] | Antibody against Le^{x}, LTA |
| | β-haptoglobin | ↑ sialyl Le^{x} | 2D PAGE and LC | [21] | Antibody against sLe^{x}, SNA, TJA-I, MAA, anti-α2-3-linked sialic acid antibody (i.e. HYB4i.e. i.e. HYB4), Siglec 1, Siglec 4 or Siglec 8 |
| | β-haptoglobin | ↑ α2-6Neu5Ac | 2D PAGE and LC | [21] | SNA, TJA-I |
| | β-haptoglobin | ↓ α2-3Neu5Ac | 2D PAGE and LC | [21] | MAA, anti-α2-3-linked sialic acid antibody (i.e. HYB4i.e. i.e. HYB4), , |
| | | | | | Siglec 1, Siglec 4 or Siglec 8 |
| | β-haptoglobin | ↑ tri-, tetra-antennary glycans | LTA affinity separation AND PAGE | [22] | DBA, PHA-E, PHA-L |
| | β-haptoglobin | ↑ α2-3Neu5Ac | LTA affinity separation AND PAGE | [22] | MAA, anti-α2-3-linked sialic acid antibody (i.e. HYB4i.e. i.e. HYB4), , Siglec 1, Siglec 4 or Siglec 8 |
| | β-haptoglobin | ↓ α2-6Neu5Ac | LTA affinity separation AND PAGE | [22] | SNA, TJA-I |
| | β-haptoglobin | ↑ antennary fucose | LTA affinity separation AND PAGE | [22] | TJA II, AAL, UEA-I, LCA, PSL, AAA, AAL |
| | β-haptoglobin | ↓ bi-antennary glycans | Con A | [22] | NPA, GNA |
| | β-haptoglobin | ↑ α2-3Neu5Ac | MAA | [22] | anti-α2-3-linked sialic acid antibody (i.e. HYB4i.e. i.e. HYB4), , Siglec 1, Siglec 4 or Siglec 8 |
| | α-1-antitrypsin | ↑ tetra-antennary glycans | CE | [20] | DBA, PHA-E, PHA-L |
| | α-1-antitrypsin | ↑ Le^{x} | CE | [20] | Antibody against Le^{x}, LTA |
| | α-1-antitrypsin | ↓ tri-, tetra-antennary glycans | LTA affinity separation AND PAGE | [22] | DBA, PHA-E, PHA-L |
| | α-1-antitrypsin | ↓ α2-3Neu5Ac | LTA affinity separation AND PAGE | [22] | MAA, anti-α2-3-linked sialic acid |
| | | | | | antibody (i.e. HYB4i.e. i.e. HYB4), , Siglec 1, Siglec 4 or Siglec 8 |
| | α-1-antitrypsin | ↑ α2-6Neu5Ac | LTA affinity separation AND PAGE | [22] | SNA, TJA-I |
| | α-1-antitrypsin | ↑ core fucose | LTA affinity separation AND PAGE | [22] | AOL, PhoSL |
| | α-1-antitrypsin | ↑ bi-antennary glycans | Con A | [22] | NPA, GNA |
| | α-1-antitrypsin | ↑ α2-6Neu5Ac | SNA | [22] | TJA-I, SCA |
| | α-1-antitrypsin | ↓ α2-3Neu5Ac | MAA | [22] | anti-α2-3-linked sialic acid antibody (i.e. HYB4i.e. i.e. HYB4), , Siglec 1, Siglec 4 or Siglec 8 |
| | α-1-antichymotrypsin | ↑ tetra-antennary glycans | CE | [20] | DBA, PHA-E, PHA-L |
| | α-1-antichymotrypsin | ↑ Le^{x} | CE | [20] | Antibody against Le^{x}, LTA |
| | α-1-antichymotrypsin | ↑ sialyl Le^{x} | 2D PAGE and LC | [21] | Antibody against sLe^{x}, SNA, TJA-I, MAA, anti-α2-3-linked sialic acid antibody (i.e. HYB4i.e. i.e. HYB4), , Siglec 1, Siglec 4 or Siglec 8 |
| | α-1-antichymotrypsin | ↑ α2-6Neu5Ac | 2D PAGE and LC | [21] | SNA, TJA-I |
| | α-1-antichymotrypsin | ↓ α2-3Neu5Ac | 2D PAGE and LC | [21] | MAA, anti-α2-3-linked sialic acid antibody (i.e. HYB4i.e. i.e. HYB4), , Siglec 1, Siglec 4 or Siglec 8 |
| | transferrin | ↓ tri-antennary glycans | CE | [20] | DBA, PHA-E, PHA-L |
| | hemopexin | ↑ Le^{x} | CE | [20] | Antibody against Le^{x}, LTA |
| | IgG | ↓ galactose | 2D PAGE and LC | [21] | RCA, RCA120, ABA, Jacalin (DSA), AlloA, ECL, PNA |
| | IgG | ↓ sialic acid | 2D PAGE and LC | [21] | SNA, TJA-I, MAA, anti-α2-3-linked sialic acid antibody (i.e. HYB4i.e. i.e. HYB4), , Siglec 1, Siglec 4 or Siglec 8 |
| | CA125 (MUC16) | ↑ sialyl Tn antigen | VVA lectin after sialidase detection by | [23] | SNA, TJA-I, MAA, anti-α2-3-linked sialic acid antibody (i.e. HYB4i.e. i.e. HYB4), , Siglec 1, Siglec 4 or Siglec 8 |
| | CA125 (MUC16) | ↑ sialyl T antigen | anticarbohydrate IgM antibodies 3C9 after sialidase detection | [23] | SNA, TJA-I, MAA, anti-α2-3-linked sialic acid antibody (i.e. HYB4i.e. i.e. HYB4), , Siglec 1, Siglec 4 or Siglec 8 |
| | CA15-3 (MUC1) | ↑ sialyl Tn antigen | VVA lectin after sialidase detection | [23] | SNA, TJA-I, MAA, anti-α2-3-linked sialic acid antibody (i.e. HYB4), Siglec 1, Siglec 4 or Siglec 8 |
| | CA15-3 (MUC1) | ↑ core fucose | PAGE/LC | [24] | PhoSL, AOL |
| | CA15-3 (MUC1) | ↑ bi-antennary glycans | PAGE/LC | [24] | Con A |
| | CA15-3 (MUC1) | ↓ tri-, tetra-antennary glycans | PAGE/LC | [24] | PHA-E, PHA-L, DBA |
| | CA15-3 (MUC1) | ↑ antennary fucose | PAGE/LC | [24] | AAL, TJA II, UEA-I, LCA, PSL, AAA, LTA |
| | human epididymis protein 4 (HE4) | ↑ Le^{y} antigen | Antibody against Lewis^{y} glycan | [25] | UEA-I |
| | Clusterin | ↑ α2-6Neu5Ac | SNA | [26] | TJA-I, SCA |
| | leucine-rich α-2-glycoprotein | ↑ α2-6Neu5Ac | SNA | [26] | TJA-I, SCA |
| | | | | | |
| **Breast** | CA15-3 (MUC1) | ↑ sulfated core1 glycan | Galectin 4 | [27] | SBA, ABA, VVA, Jacalin (DSA), BPL, PNA, GSL1, SJA |
| | CA15-3 (MUC1) | ↑ Tn, sialyl Tn antigens | | [28] | SBA, DBA, VVA, SNA, SNA, TJA-I, MAA, anti-α2-3-linked sialic acid antibody (i.e. HYB4) |
| | CA15-3 (MUC1) | change sialyl T, Tn antigens | LC | [29] | SNA, TJA-I, MAA, anti-α2-3-linked sialic acid antibody (i.e. HYB4) , Siglec 1, Siglec 4 or Siglec 8; SBA, ABA, VVA, BPL, Jacalin, PNA |
| | CA15-3 (MUC1) | change α2-8Neu5Ac | LC | [29] | antibody against poly(sialic acid), Siglec 7 or Siglec 11 |
| | CA15-3 (MUC1) | change in sialylation | LC | [29] | SNA, TJA-I, MAA, anti-α2-3-linked sialic acid antibody (i.e. HYB4), Siglec 1, Siglec 4 or Siglec 8 |
| | CA15-3 (MUC1) | change in core 2 glycan | LC | [29] | RCA, RCA120, ABA, Jacalin (DSA), PNA, WGA |
| | CA15-3 | change in sialylation | MAA | [30] | SNA, TJA-I, MAA, anti-α2-3-linked sialic acid antibody (i.e. HYB4), Siglec 1, Siglec 4 or Siglec 8 |
| | CA15-3 (MUC1) | change in sialylation | MAA, SNA, TVA=WGA | [30] | SNA, TJA-I, MAA, anti-α2-3-linked sialic acid antibody (i.e. HYB4), Siglec 1, Siglec 4 or Siglec 8 |
| | CA27.29 | change in sialylation | MAA | [30] | SNA, TJA-I, MAA, anti-α2-3-linked sialic acid antibody (i.e. HYB4), , Siglec 1, Siglec 4 or Siglec 8 |
| | HER2 | change in antennary fucose | UEA | [30] | TJA II, AAL, LCA, PSL, AAA, LTA |
| | HER2 | change in sialylation | MAA, SNA, TVA=WGA | [30] | SNA, TJA-I, MAA, anti-α2-3-linked sialic acid antibody (i.e. HYB4), , Siglec 1, Siglec 4 or Siglec 8 |
| | CEA | change in tri-, tetra-antennary glycans | | [31] | PHA-E, PHA-L, DBA |
| | | | | | |
| **Colorectal** | β-haptoglobin | ↑ antennary fucose | AAL | [32] | TJA II, UEA-I, LCA, PSL, AAA, LTA |
| | β-haptoglobin | ↑ antennary fucose | AAL | [33] | TJA II, UEA-I, LCA, PSL, AAA, LTA |
| | β-haptoglobin | ↑ bi-antennary glycans | PHA-E | [32] | Con A, PHA-L, DBA |
| | β-haptoglobin | ↑ antennary/cor e fucose | AAL, AOL, LTA | [34] | TJA II, UEA-I, LCA, PSL, AAA, PhoSL |
| | β-haptoglobin | ↑ dimer: Le^{a} on Le^{a} | mouse monoclonal antibody NCC-ST-421, | [35] | |
| | β-haptoglobin | ↑ Galβ1-4GIcNAc | Galectin 3 | [36] | ECA, AlloA |
| | Carcinoembryonic antigen (CEA) | ↑ Le^{x} | LTA, Antibody against sialyl Lewis^{x} glycan | [37] | |
| | CEA | ↑ Le^{y} | UEA-I, Antibody against sialyl Lewis^{y} glycan | [37] | |
| | CEA | ↑ α2-3Neu5Ac | MAA | [37] | anti-α2-3-linked sialic acid antibody (i.e. HYB4), Siglec 1, Siglec 4 or Siglec 8 |
| | CEA | ↑ α -D-Man | NPA | [37] | Con A, GNA |
| | CEA | ↑ tri-, tetra-antennary glycans | PHA-L | [37] | PHA-E, DBA |
| | CEA | ↑ mannose, fucose | DC-SIGN | [37] | NPA, Con A, GNA, AAL, TJA II, UEA-I, LCA, PSL, AAA, LTA, AOL, PhoSL |
| | CEA | ↓ terminal GalNAc | MGBL | [37] | DBA, SBA, VVA, HPA, WFA |
| | CEA | ↑ Gal · 1-4GIcNAc | Galectin 3 | | |
| | CA 19-9 (MUC1) | ↑ T antigen | SBA | [37] | ABA |
| | CA 19-9 (MUC1) | ↑ Galβ1-3GalNAc | PNA | [37] | ABA, Jacalin |
| | CA 19-9 (MUC1) | ↑ antennary fucose | UEA | [37] | TJA II, AAL, LCA, PSL, AAA, LTA |
| | CA 19-9 (MUC1) | ↑ α2-3Neu5Ac | MAA | [37] | anti-α2-3-linked sialic acid antibody (i.e. HYB4), Siglec 1, Siglec 4 or Siglec 8 |
| | CA 19-9 (MUC1) | ↑ α2-6Neu5Ac | SNA | [37] | TJA-I |
| | CA 19-9 (MUC1) | ↓ tri-, tetra-antennary glycans | PHA-E, PHA-L | [37] | DBA |
| | CA 19-9 (MUC1) | ↑ terminal GalNAc | MGBL | [37] | DBA, SBA, HPA, WFA |
| | Complement C3 (UniProtKB: P01024) | ↑ antennary fucose | AAL | [38] | TJA II, UEA-I, LCA, PSL, AAA, LTA |
| | Complement C3 (UniProtKB: P01024) | ↑ Gal β 1-3GalNAc | PNA | [38] | ABA, Jacalin |
| | Complement C3 (UniProtKB: P01024) | ↑ α2-3Neu5Ac | MAA | [38] | anti-α2-3-linked sialic acid antibody (i.e. HYB4), Siglec 1, Siglec 4 or Siglec 8 |
| | Complement C3 (UniProtKB: P01024) | ↑ α2-6Neu5Ac | SNA | [38] | TJA-I |
| | Kininogen-I (UniProtKB: P01042) | ↑ high mannose | Con A | [38] | NPA, GNA |
| | Kininogen-I (UniProtKB: P01042) | ↑ antennary fucose | AAL | [38] | TJA II, UEA-I, LCA, PSL, AAA, LTA |
| | Kininogen-I (UniProtKB: P01042) | ↑ Gal*β* 1-3GalNAc | PNA | [38] | ABA, Jacalin |
| | Kininogen-I (UniProtKB: P01042) | ↑ α2-3Neu5Ac | MAA | [38] | anti-α2-3-linked sialic acid antibody (i.e. HYB4), Siglec 1, Siglec 4 or Siglec 8 |
| | Kininogen-I(UniProtKB: P01042) | ↑ α2-6Neu5Ac | SNA | [38] | TJA-I |
| | Histidine-rich glycoprotein (UniProtKB: P04196) | ↑ antennary fucose | AAL | [38] | TJA II, UEA-I, LCA, PSL, AAA, LTA |
| | Histidine-rich glycoprotein (UniProtKB: P04196) | ↑ α2-6Neu5Ac | SNA | [38] | TJA-I |
| | | | | | |
| **Pancreati c** | α₁-β-glycoprotein | ↑ Neu5Ac | SNA | [39] | TJA-I, anti-α2-3-linked sialic acid antibody (i.e. HYB4), Siglec 1, Siglec 4 or Siglec 8 |
| | Amyloid p-component | ↑ Neu5Ac | SNA | [39] | TJA-I, anti-α2-3-linked sialic acid antibody (i.e. HYB4), Siglec 1, Siglec 4 or Siglec 8 |
| | β-2-glycoprotein 1 (P02749) | ↑ antennary fucose | AAL | [40] | TJA II, UEA-I, LCA, PSL, AAA, LTA |
| | β-2-glycoprotein 1 (UniProtKB: P02749) | ↑ α2-3Neu5Ac | MAA | [40] | anti-α2-3-linked sialic acid antibody (i.e. HYB4), Siglec 1, Siglec 4 or Siglec 8 |
| | β-2-glycoprotein 1 (UniProtKB: P02749) | ↑ α2-6Neu5Ac | SNA | [40] | TJA-I |
| | β-2-glycoprotein 1 (UniProtKB: P02749) | ↑ high mannose | Con A | [40] | NPA, GNA |
| | β-2-glycoprotein 1 (UniProtKB: P02749) | ↑ Gal*β* 1-3GalNAc | PNA | [40] | ABA, Jacalin |
| | hemopexin (UniProtKB: P02790) | ↑ antennary fucose | AAL | [40] | TJA II, UEA-I, LCA, PSL, AAA, LTA |
| | hemopexin (UniProtKB: P02790) | ↑ α2-3Neu5Ac | MAA | [40] | anti-α2-3-linked sialic acid antibody (i.e. HYB4), Siglec 1, Siglec 4 or Siglec 8 |
| | hemopexin (UniProtKB: P02790) | ↑ α2-6Neu5Ac | SNA | [40] | TJA-I |
| | hemopexin (UniProtKB: P02790) | ↑ high mannose | Con A | [40] | NPA, GNA |
| | haptoglobin-related protein (UniProtKB: P00739) | ↑ antennary fucose | AAL | [40] | TJA II, UEA-I, LCA, PSL, AAA, LTA |
| | haptoglobin-related protein (UniProtKB: P00739) | ↑ α2-3Neu5Ac | MAA | [40] | anti-α2-3-linked sialic acid antibody (i.e. HYB4), Siglec 1, Siglec 4 or Siglec 8 |
| | haptoglobin-related protein (UniProtKB: P00739) | ↑ α2-6Neu5Ac | SNA | [40] | TJA-I |
| | haptoglobin-related protein (UniProtKB: P00739) | ↑ high mannose | Con A | [40] | NPA, GNA |
| | haptoglobin-related protein (UniProtKB: P00739) | ↑ Gal*β* 1-3GalNAc | PNA | [40] | ABA, Jacalin |
| | serum amyloid P-component (UniProtKB: P02743) | ↑ antennary fucose | AAL | [40] | TJA II, UEA-I, LCA, PSL, AAA, LTA |
| | serum amyloid P-component (UniProtKB: P02743) | ↑ α2-3Neu5Ac | MAA | [40] | anti-α2-3-linked sialic acid antibody (i.e. HYB4), Siglec 1, Siglec 4 or Siglec 8 |
| | serum amyloid P-component (UniProtKB: P02743) | ↑ α2-6Neu5Ac | SNA | [40] | TJA-I |
| | serum amyloid P-component (UniProtKB: P02743) | ↑ high mannose | Con A | [40] | NPA, GNA |
| | serum amyloid P-component (UniProtKB: P02743) | ↑ Gal *β* 1-3GalNAc | PNA | [40] | ABA, Jacalin (DSA) |
| | clusterin (UniProtKB: P10909) | ↑ antennary fucose | AAL | [40] | TJA II, UEA-I, LCA, PSL, AAA, LTA |
| | clusterin (UniProtKB: P10909) | ↑ α2-3Neu5Ac | MAA | [40] | anti-α2-3-linked sialic acid antibody (i.e. HYB4), Siglec 1, Siglec 4 or Siglec 8 |
| | clusterin (UniProtKB: P10909) | ↑ α2-6Neu5Ac | SNA | [40] | TJA-I |
| | clusterin (UniProtKB: P10909) | ↑ Gal*β* 1-3GalNAc | PNA | [40] | ABA, Jacalin |
| | antithrombin-III (UniProtKB: P01008) | ↑ antennary fucose | AAL | [40] | TJA II, UEA-I, LCA, PSL, AAA, LTA |
| | antithrombin-III (UniProtKB: P01008) | ↑ α2-3Neu5Ac | MAA | [40] | anti-α2-3-linked sialic acid antibody (i.e. HYB4), Siglec 1, Siglec 4 or Siglec 8 |
| | antithrombin-III (UniProtKB: P01008) | ↑ α2-6Neu5Ac | SNA | [40] | TJA-I |
| | antithrombin-III (UniProtKB: P01008) | ↑ high mannose | Con A | [40] | NPA, GNA |
| | antithrombin-III (UniProtKB: P01008) | ↑ Gal*β* 1-3GalNAc | PNA | [40] | ABA, Jacalin (DSA) |
| | kininogen-1 (UniProtKB: P01042) | ↑ antennary fucose | AAL | [40] | TJA II, UEA-I, LCA, PSL, AAA, LTA |
| | kininogen-1 (UniProtKB: P01042) | ↑ α2-3Neu5Ac | MAA | [40] | anti-α2-3-linked sialic acid antibody (i.e. HYB4), Siglec 1, Siglec 4 or Siglec 8 |
| | kininogen-1 (UniProtKB: P01042) | ↑ α2-6Neu5Ac | SNA | [40] | TJA-I |
| | kininogen-1 (UniProtKB: P01042) | ↑ high mannose | Con A | [40] | NPA, GNA |
| | kininogen-1 (UniProtKB: P01042) | ↑ Gal β 1-3GalNAc | PNA | [40] | ABA, Jacalin (DSA) |
| | plasma protease C1 inhibitor (UniProtKB: P05155) | ↑ α2-6Neu5Ac | SNA | [40] | TJA-I |
| | β-haptoglobin | ↑ antennary fucose | AAL | [41] | TJA II, UEA-I, LCA, PSL, AAA, LTA |
| | β-haptoglobin | ↑ antennary fucose | AAL | [42] | TJA II, UEA-I, LCA, PSL, AAA, LTA |
| | β-haptoglobin | ↑ core fucose | AOL | [41] | PhoSL |
| | β-haptoglobin | ↑ core fucose | PhoSL | [43] | AOL |
| | α-1-antichymotrypsin | ↑ antennary fucose | AAL | [42] | TJA II, UEA-I, LCA, PSL, AAA, LTA |
| | thrombospondin-1 | ↑ antennary fucose | AAL | [42] | TJA II, UEA-I, LCA, PSL, AAA, LTA |
| | α-1-antitrypsin | ↑ antennary fucose | AAL | [42] | TJA II, UEA-I, LCA, PSL, AAA, LTA |
| | Mucin (CAM 17.1) | ↑ *β* -D-GlcNAc, Neu5Ac | WGA | [44, 45] | DSA, LEL, SNA, TJA-I |
| | MUC16 | ↑ antennary fucose | AAL | [46, 47] | TJA II, UEA-I, LCA, PSL, AAA, LTA |
| | MUC16 | ↓ T antigen | BPL, Jacalin (DSA), PNA | [46] | SBA, VVA, ABA, GSL1, SJA |
| | MUC16 | ↓ Gal-GIcNAc | ECL, PHA-L | [46] | PHA-E, AlloA, ECA, |
| | MUC16 | ↓ GalNAc | DBA, GSL1, SBA, VVL, SJA | [46] | ABA, BPL, PNA |
| | MUC16 | ↓ GlcNAc | GSL2, STL | [46] | DSA, LEL, WGA |
| | MUC16 | ↓ mannose | Con A | [46] | GNA, NPA |
| | MUC5ac | ↑ T antigen | Jacalin | [46] | SBA, ABA, VVA, BPL, PNA |
| | MUC5ac | ↑ antennary fucose | AAL | [46] | TJA II, EA-I, LCA, PSL, AAA, LTA |
| | MUC5ac | ↑ T antigen | Jacalin (DSA) | [46] | SBA, ABA, VVA, BPL, PNA, GSL1, SJA |
| | MUC5ac | ↓ Gal-GIcNAc | ECA, PHA-L, RCA120 | [46] | PHA-E, RCA |
| | MUC5ac | ↓ GalNAc | DBA, VVA, SJA | [46] | GSL1, SBA, ABA, BPL, PNA |
| | MUC5ac | ↓ GlcNAc | GSL 2, LEL, STL | [46] | DSA, LEL, WGA, GSL2, STL |
| | MUC1 | ↓ Gal-GlcNAc, tetra-antennary glycans | PHA-L | [46] | ECA, PHA-L, RCA120, PHA-E, RCA; DBA |
| | MUC1 | ↓ T antigen | Jacalin (DSA) | [46] | SBA, ABA, VVA, BPL, PNA, GSL1, SJA |
| | MUC1 | ↓ GalNAc | DBA | [46] | VVA, SJA, GSL1, SBA, ABA, BPL, PNA |
| | MUC1 | ↑ Gal α 1-3Gal | GSL 1 | [46] | |
| | MUC1 | ↓ GlcNAc | GSL 2, LEL, STL | [46] | DSA, LEL, WGA, GSL2, STL |
| | | | | | |
| Thyroid | Thyroglobulin (TG) | ↓ antennary fucose | LCA | [48, 49] | TJA II, AAL, UEA-I, PSL, AAA, LTA |
| | TG | ↑ terminal galactose | RCA | [50] | RCA120, ABA, AlloA, Jacalin (DSA), ECL, PNA |
| | TG | ↑ Gal-GIcNAc | LC assays | [50] | ECA, PHA-L, RCA120, PHA-E, RCA |
| | TG | ↑ tri-antennary glycans | LC assays | [50] | PHA-E, PHA-L, DBA |
| | TG | ↑ antennary fucose | LC assays | [50] | TJA II, AAL, UEA-I, LCA, PSL, AAA, LTA |
| | TG | ↑ mannose | LC assays | [50] | Con A, NPA, GNA |
| | | | | | |
| **Liver** | α₁-antitrypsin (AAT) | ↑ antennary fucose | LCA | [51] | TJA II, UEA-I, AAL, PSL, AAA, LTA |
| | α₁-antitrypsin (AAT) | ↑ antennary fucose | AAL | [52, 53] | TJA II, UEA-I, LCA, PSL, AAA, LTA |
| | α-fetoprotein (AFP) | ↑ antennary fucose | LCA | [51, 54] | TJA II, AAL, UEA-I, PSL, AAA, LTA |
| | α-fetoprotein (AFP) | ↑ antennary fucose | AAL | [54] | TJA II, UEA-I, LCA, PSL, AAA, LTA |
| | AFP-L3 | ↑ antennary fucose | LCA | [55, 56] | TJA II, UEA-I, PSL, AAA, LTA |
| | transferrin | ↑ antennary fucose | LCA | [51] | TJA II, UEA-I, PSL, AAA, LTA |
| | α₁-antichymotrypsin (AAT) | ↑ antennary fucose | AAL | [52] | TJA II, UEA-I, LCA, PSL, AAA, LTA |
| | α-1-acid glycoprotein 1 | ↑ antennary fucose | AAL | [52] | TJA II, UEA-I, LCA, PSL, AAA, LTA |
| | ceruloplasmin | ↑ antennary fucose | AAL | [52] | TJA II, UEA-I, LCA, PSL, AAA, LTA |
| | α-2-macroglobulin | ↑ antennary fucose | AAL, LCA | [54] | TJA II, UEA-I, PSL, AAA, LTA |
| | α-2-HS-glycoprotein | ↑ antennary fucose | AAL | [53] | TJA II, UEA-I, LCA, PSL, AAA, LTA |
| | Fetuin A | ↑ antennary fucose | AAL | [57] | TJA II, UEA-I, LCA, PSL, AAA, LTA |
| | hemopexin | ↑ antennary fucose | AAL | [54, 57] | TJA II, UEA-I, LCA, PSL, AAA, LTA |
| | hemopexin | ↑ antennary fucose | LCA | [54] | TJA II, AAL, UEA-I, PSL, AAA, LTA |
| | Ceruloplasmin | ↑ antennary fucose | AAL, LCA | [58] | TJA II, UEA-I, PSL, AAA, LTA |
| | C3 complement | ↑ antennary fucose | AAL, LCA | [58] | TJA II, UEA-I, PSL, AAA, LTA |
| | Histidine rich glycoprotein | ↑ antennary fucose | AAL, LCA | [58] | TJA II, UEA-I, PSL, AAA, LTA |
| | Monocyte differentiation antigen CD14 | ↑ antennary fucose | AAL, LCA | [58] | TJA II, UEA-I, PSL, AAA, LTA |
| | Hepatocyte growth factor activator | ↑ antennary fucose | AAL, LCA | [58] | TJA II, UEA-I, PSL, AAA, LTA |
| | | | | | |
| **Lung** | β-haptoglobin | ↑ antennary fucose | AAL | [59] | TJA II, UEA-I, PSL, AAA, LCA, LTA |
| | β-haptoglobin | ↑ antennary fucose | AAL | [59] | TJA II, UEA-I, PSL, AAA, LCA, LTA |
| | β-haptoglobin | ↑ antennary fucose | MS | [60] | AAL, TJA II, UEA-I, LCA, PSL, AAA, LTA |
| | β-haptoglobin | ↑ core fucose | MS | [60] | AOL, PhoSL |
| | β-haptoglobin | ↑ tri-, tetra-antennary glycans | MS | [60] | PHA-E, PHA-L, DBA |
| | β-haptoglobin | ↑ α2-6Neu5Ac | MS | [61] | SNA, TJA-I |
| | β-haptoglobin | ↑ antennary fucose | MS | [61] | AAL, TJA II, UEA-I, LCA, PSL, AAA, LTA |
| | β-haptoglobin | ↑ sialyl Le^{x} | LC | [62] | SNA, TJA-I, MAA, anti-α2-3-linked sialic acid antibody (i.e. HYB4), Siglec 1, Siglec 4 or Siglec 8 |
| | β-haptoglobin | ↑ tri-antennary | LC | [62] | PHA-E, PHA-L, DBA |
| | β-haptoglobin | ↑ sialic acid | LC | [62] | SNA, TJA-I, MAA, anti-α2-3-linked sialic acid antibody (i.e. HYB4), Siglec 1, Siglec 4 or Siglec 8 |
| | fibronectin | ↑ Galβ1-3GalNAc | PNA | [63] | ABA, Jacalin (DSA) |
| | α₁-acid glycoprotein | ↑ antennary fucose | AAL | [64] | TJA II, UEA-I, LCA, PSL, AAA, LTA |
| | α₁-acid glycoprotein | ↑ sialyl Le^{x} | Antibody against sLe^{x} | [64] | SNA, TJA-I, MAA, anti-α2-3-linked sialic acid antibody (i.e. HYB4), Siglec 1, Siglec 4 or Siglec 8 |
| | α-1-antitrypsin | ↑ antennary fucose | AAL | [65] | TJA II, UEA-I, LCA, PSL, AAA, LTA |
| | α-1-antitrypsin | ↑ β-Gal, Galβ1-4GIcNAc | RCA120 | [65] | RCA, ECL, AlloA |
| | α-1-antitrypsin | ↑ α-Gal and α-GalNAc | BS-I | [65] | DBA, SBA, HPA |
| | α-1-antitrypsin | ↑ (GlcNAc)ₙ | WGA | [65] | LEL |
| | α-1-antitrypsin | ↑ Branched (LacNAc)ₙ | PWM | [65] | |
| | α-1-antitrypsin | ↑ high-mannose, Manα1-3Man | GNA | [65] | Con A, NPA |
| | | | | | |
| **Stomach** | α₁-acid glycoprotein | ↑ bi-antennary glycans | Con A | [66] | NPA, GNA |
| | α₁-acid glycoprotein | ↓ galactose | | [66] | RCA, RCA120, ABA, AlloA, Jacalin (DSA), ECL, PNA |
| | α₁-acid glycoprotein | ↑ Le^{x} | | [66] | LTA |
| | β-haptoglobin | ↑ sialyl Le^{x} (sLe^{x}) | anti-sLe^{X} mouse monoclonal KM93 antibody | [67] | SNA, TJA-I, MAA, anti-α2-3-linked sialic acid antibody (i.e. HYB4), Siglec 1, Siglec 4 or Siglec 8 |
| | β-haptoglobin | ↑ tri-, tetra-antennary glycans | LC/MS | [68] | PHA-E, PHA-L, DBA |
| | β-haptoglobin | ↑ antennary fucose | LC/MS | [68] | AAL, TJA II, UEA-I, LCA, PSL, AAA, LTA |
| | β-haptoglobin | ↑ sialyl-Le^{a} (sLe^{a}) | LC/MS | [68] | Antibody against sLe^{a}, SNA, TJA-I, MAA, anti-α2-3-linked sialic acid antibody (i.e. HYB4), Siglec 1, Siglec 4 or Siglec 8 |
| | β-haptoglobin | ↑ sialyl-Le^{a} (sLe^{a}) | LC/MS | [68] | Antibody against sLe^{a}, SNA, TJA-I, MAA, anti-α2-3-linked sialic acid antibody (i.e. HYB4) |
| | β-haptoglobin | ↑ antennary fucose | AAL | [68] | TJA II, UEA-I, LCA, PSL, AAA, LTA |
| | β-haptoglobin | ↑ (GlcNAc)ₙ | WGA | [68] | LEL |
| | β-haptoglobin | ↓ high mannose | Con A | [68] | NPA, GNA |
| | leucine-rich-α2- | ↑ sialyl Le^{x} (sLe^{x}) | anti-sLe^{X} mouse monoclonal KM93 antibody | [67] | Antibody against sLe^{a}, SNA, TJA-I, MAA, anti-α2-3-linked sialic acid antibody (i.e. HYB4), Siglec 1, Siglec 4 or Siglec 8 |
| | glycoprotein | | | | |
| | | | | | |
| **Testicular** | Human chorionic gonadotropin-*β* | ↑ fucose | LC-MS | [69] | AAL, TJA II, UEA-I, LCA, PSL, AAA, LTA, PhoSL, AOL |
| | Human chorionic gonadotropin-*β* | ↑ tri-antennary glycans | LC-MS | [69] | PHA-E, PHA-L, DBA |
| | AFP-L3 | ↑ antennary fucose | LCA | [70] | AAL, TJA II, UEA-I, LCA, PSL, AAA, LTA |
| | | | | | |
| **Bladder** | MUC1 | ↑ antennary fucose | AAL | [71, 72] | TJA II, UEA-I, LCA, PSL, AAA, LTA |
| | endoplasmin (HSP90B1) | ↑ antennary fucose | AAL | [71, 72] | TJA II, UEA-I, LCA, PSL, AAA, LTA |
| | Golgi apparatus protein 1 (GLG1) | ↑ antennary fucose | AAL | [71, 72] | TJA II, UEA-I, LCA, PSL, AAA, LTA |
| | prostatic acid phosphatase (ACPP) | ↑ antennary fucose | AAL | [71, 72] | TJA II, UEA-I, LCA, PSL, AAA, LTA |
| | Ig gamma-2 chain C region (IGHG2) | ↑ antennary fucose | AAL | [71, 72] | TJA II, UEA-I, LCA, PSL, AAA, LTA |
| | deoxyribonuclease -2-alpha (DNASE2A) | ↑ antennary fucose | AAL | [71, 72] | TJA II, UEA-I, LCA, PSL, AAA, LTA |
| | integrin | ↑ sialic acid | MS | [73] | SNA, TJA-I, MAA, anti-α2-3-linked sialic acid antibody (i.e. HYB4), Siglec 1, Siglec 4 or Siglec 8 |
| | integrin | ↑ tetra-antennary glycans | MS | [73] | PHA-E, PHA-L, DBA |
| | MUC16 | ↑ sialyl Tn | LC/MS | [74] | SNA, TJA-I, MAA, anti-α2-3-linked sialic acid antibody (i.e. HYB4) |
| | α-1-antitrypsin | ↑ high mannose | Con A | [75] | NPA, GNA |
| | α-1-antitrypsin | ↑ (GlcNAcβ1-4)ₙ | WGA | [75] | LEL |

### References as shown in Table 1 are as follows:

[1] C. Ohyama, M. Hosono, K. Nitta, M. Oh-eda, K. Yoshikawa, T. Habuchi, Y. Arai, M. Fukuda, Carbohydrate structure and differential binding of prostate specific antigen to Maackia amurensis lectin between prostate cancer and benign prostate hypertrophy, Glycobiology, 14 (2004) 671-679.
[2] K. Fukushima, T. Satoh, S. Baba, K. Yamashita, α1,2-Fucosylated and β-N-acetylgalactosaminylated prostate-specific antigen as an efficient marker of prostatic cancer, Glycobiology, 20 (2010) 452-460.
[3] T. Ishikawa, T. Yoneyama, Y. Tobisawa, S. Hatakeyama, T. Kurosawa, K. Nakamura, S. Narita, K. Mitsuzuka, W. Duivenvoorden, J.H. Pinthus, Y. Hashimoto, T. Koie, T. Habuchi, Y. Arai, C. Ohyama, An Automated Micro-Total Immunoassay System for Measuring Cancer-Associated 2,3-linked Sialyl N-Glycan-Carrying Prostate-Specific Antigen May Improve the Accuracy of Prostate Cancer Diagnosis, Int. J. Mol. Sci., 18 (2017) 15.
[4] D. Pihikova, P. Kasak, P. Kubanikova, R. Sokol, J. Tkac, Aberrant sialylation of a prostate-specific antigen: Electrochemical label-free glycoprofiling in prostate cancer serum samples, Anal. Chim. Acta, 934 (2016) 72-79.
[5] C. Ohyama, T. Koie, T. Yoneyama, Y. Tobisawa, Quantification of prostate cancer-associated aberrant glycosylation of prostate-specific antigen, Glycoscience: Biology and Medicine, Springer2015, pp. 1373-1377.
[6] E. Llop, M. Ferrer-Batalle, S. Barrabes, P.E. Guerrero, M. Ramirez, R. Saldova, P.M. Rudd, R.N. Aleixandre, J. Comet, R. de Llorens, R. Peracaula, Improvement of Prostate Cancer Diagnosis by Detecting PSA Glycosylation-Specific Changes, Theranostics, 6 (2016) 1190-1204.
[7] M. Ferrer-Batalle, E. Llop, M. Ramirez, R.N. Aleixandre, M. Saez, J. Comet, R. de Llorens, R. Peracaula, Comparative Study of Blood-Based Biomarkers, alpha 2,3-Sialic Acid PSA and PHI, for High-Risk Prostate Cancer Detection, International Journal of Molecular Sciences, 18 (2017) 12.
[8] T. Yoneyama, C. Ohyama, S. Hatakeyama, S. Narita, T. Habuchi, T. Koie, K. Mori, K.I. Hidari, M. Yamaguchi, T. Suzuki, Measurement of aberrant glycosylation of prostate specific antigen can improve specificity in early detection of prostate cancer, Biochem. Biophys. Res. Commun., 448 (2014) 390-396.
[9] N. Idil, I. Percin, V. Karakoc, H. Yavuz, N. Aksoz, A. Denizli, Concanavalin A immobilized magnetic poly(glycidyl methacrylate) beads for prostate specific antigen binding, Colloid Surf. B-Biointerfaces, 134 (2015) 461-468.
[10] M.V. Dwek, A. Jenks, A.J. Leathem, A sensitive assay to measure biomarker glycosylation demonstrates increased fucosylation of prostate specific antigen (PSA) in patients with prostate cancer compared with benign prostatic hyperplasia, Clin. Chim. Acta, 411 (2010) 1935-1939.
[11] K. Hagiwara, Y. Tobisawa, T. Kaya, T. Kaneko, S. Hatakeyama, K. Mori, Y. Hashimoto, T. Koie, Y. Suda, C. Ohyama, T. Yoneyama, Wisteria floribunda Agglutinin and Its Reactive-Glycan-Carrying Prostate-Specific Antigen as a Novel Diagnostic and Prognostic Marker of Prostate Cancer, Int. J. Mol. Sci., 18 (2017) 16.
[12] T. Kaya, T. Kaneko, S. Kojima, Y. Nakamura, Y. Ide, K. Ishida, Y. Suda, K. Yamashita, High-sensitivity immunoassay with surface plasmon field-enhanced fluorescence spectroscopy using a plastic sensor chip: Application to quantitative analysis of total prostate-specific antigen and GalNAcβ1-4GlcNAc-linked prostate-specific antigen for prostate cancer diagnosis, Anal. Chem., 87 (2015) 1797-1803.
[13] Q.K. Li, L. Chen, M.-H. Ao, J.H. Chiu, Z. Zhang, H. Zhang, D.W. Chan, Serum fucosylated prostate-specific antigen (PSA) improves the differentiation of aggressive from non-aggressive prostate cancers, Theranostics, 5 (2015) 267.
[14] K. Fujita, T. Hayashi, K. Matsuzaki, W. Nakata, M. Masuda, A. Kawashima, T. Ujike, A. Nagahara, M. Tsuchiya, Y. Kobayashi, S. Nojima, M. Uemura, E. Morii, E. Miyoshi, N. Nonomura, Decreased fucosylated PSA as a urinary marker for high Gleason score prostate cancer, Oncotarget, 7 (2016) 56643-56649.
[15] S. Takahashi, T. Sugiyama, M. Shimomura, Y. Kamada, K. Fujita, N. Nonomura, E. Miyoshi, M. Nakano, Site-specific and linkage analyses of fucosylated N-glycans on haptoglobin in sera of patients with various types of cancer: possible implication for the differential diagnosis of cancer, Glycoconjugate J., 33 (2016) 471-482.
[16] S. Kazuno, T. Fujimura, T. Arai, T. Ueno, K. Nagao, M. Fujime, K. Murayama, Multi-sequential surface plasmon resonance analysis of haptoglobin-lectin complex in sera of patients with malignant and benign prostate diseases, Anal. Biochem., 419 (2011) 241-249.
[17] S.-J. Yoon, S.-Y. Park, P.-C. Pang, J. Gallagher, J.E. Gottesman, A. Dell, J.-H. Kim, S.-I. Hakomori, N-glycosylation status of β-haptoglobin in sera of patients with prostate cancer vs. benign prostate diseases, Int. J. Oncol., 36 (2010) 193-203.
[18] T. Fujimura, Y. Shinohara, B. Tissot, P.C. Pang, M. Kurogochi, S. Saito, Y. Arai, M. Sadilek, K. Murayama, A. Dell, Glycosylation status of haptoglobin in sera of patients with prostate cancer vs. benign prostate disease or normal subjects, Int. J. Cancer, 122 (2008) 39-49.
[19] K. Fujita, M. Shimomura, M. Uemura, W. Nakata, M. Sato, A. Nagahara, Y. Nakai, S. Takamatsu, E. Miyoshi, N. Nonomura, Serum fucosylated haptoglobin as a novel prognostic biomarker predicting high-Gleason prostate cancer, The Prostate, 74 (2014) 1052-1058.
[20] S. Weiz, M. Wieczorek, C. Schwedler, M. Kaup, E.I. Braicu, J. Sehouli, R. Tauber, V. Blanchard, Acute-phase glycoprotein N-glycome of ovarian cancer patients analyzed by CE-LIF, Electrophoresis, 37 (2016) 1461-1467.
[21] R. Saldova, L. Royle, C.M. Radcliffe, U.M. Abd Hamid, R. Evans, J.N. Arnold, R.E. Banks, R. Hutson, D.J. Harvey, R. Antrobus, Ovarian cancer is associated with changes in glycosylation in both acute-phase proteins and IgG, Glycobiology, 17 (2007) 1344-1356.
[22] G. Turner, M. Goodarzi, S. Thompson, Glycosylation of alpha-1-proteinase inhibitor and haptoglobin in ovarian cancer: evidence for two different mechanisms, Glycoconjugate J., 12 (1995) 211-218.
[23] K. Chen, A. Gentry-Maharaj, M. Burnell, C. Steentoft, L. Marcos-Silva, U. Mandel, I. Jacobs, A. Dawnay, U. Menon, O. Blixt, Microarray Glycoprofiling of CA125 improves differential diagnosis of ovarian cancer, J. Proteome Res., 12 (2013) 1408-1418.
[24] R. Saldova, W.B. Struwe, K. Wynne, G. Elia, M.J. Duffy, P.M. Rudd, Exploring the glycosylation of serum CA125, International journal of molecular sciences, 14 (2013) 15636-15654.
[25] H. Zhuang, J. Gao, Z. Hu, J. Liu, D. Liu, B. Lin, Co-expression of Lewis y antigen with human epididymis protein 4 in ovarian epithelial carcinoma, PLoS One, 8 (2013) e68994.
[26] J. Wu, X. Xie, S. Nie, R.J. Buckanovich, D.M. Lubman, Altered expression of sialylated glycoproteins in ovarian cancer sera using lectin-based ELISA assay and quantitative glycoproteomics analysis, J. Proteome Res., 12 (2013) 3342-3352.
[27] H. Ideo, Y. Hinoda, K. Sakai, I. Hoshi, S. Yamamoto, M. Oka, K. Maeda, N. Maeda, S. Hazama, J. Amano, Expression of mucin 1 possessing a 3'-sulfated core1 in recurrent and metastatic breast cancer, Int. J. Cancer, 137 (2015) 1652-1660.
[28] S.A. Svarovsky, L. Joshi, Cancer glycan biomarkers and their detection-past, present and future, Anal. Methods, 6 (2014) 3918-3936.
[29] S.J. Storr, L. Royle, C.J. Chapman, U.M.A. Hamid, J.F. Robertson, A. Murray, R.A. Dwek, P.M. Rudd, The O-linked glycosylation of secretory/shed MUC1 from an advanced breast cancer patient's serum, Glycobiology, 18 (2008) 456-462.
[30] H.A. Badr, D.M. AlSadek, A.A. Darwish, A.I. ElSayed, B.O. Bekmanov, E.M. Khussainova, X. Zhang, W.C. Cho, L.B. Djansugurova, C.-Z. Li, Lectin approaches for glycoproteomics in FDA-approved cancer biomarkers, Expert review of proteomics, 11 (2014) 227-236.
[31] Y. Taeda, M. Nose, S. Hiraizumi, N. Ohuchi, Expression of L-PHA-binding proteins in breast cancer: Reconstitution and molecular characterization of beta 1-6 branched oligosaccharides in three-dimensional cell culture, Breast Cancer Res. Treat., 38 (1996) 313-324.
[32] S.Y. Park, S.J. Yoon, Y.T. Jeong, J.M. Kim, J.Y. Kim, B. Bernert, T. Ullman, S.H. Itzkowitz, J.H. Kim, S.i. Hakomori, N-glycosylation status of β-haptoglobin in sera of patients with colon cancer, chronic inflammatory diseases and normal subjects, Int. J. Cancer, 126 (2010) 142-155.
[33] Y. Takeda, S. Shinzaki, K. Okudo, K. Moriwaki, K. Murata, E. Miyoshi, Fucosylated haptoglobin is a novel type of cancer biomarker linked to the prognosis after an operation in colorectal cancer, Cancer, 118 (2012) 3036-3043.
[34] S.Y. Park, S.H. Lee, N. Kawasaki, S. Itoh, K. Kang, S. Hee Ryu, N. Hashii, J.M. Kim, J.Y. Kim, J. Hoe Kim, α1-3/4 fucosylation at Asn 241 of β-haptoglobin is a novel marker for colon cancer: A combinatorial approach for development of glycan biomarkers, Int. J. Cancer, 130 (2012) 2366-2376.
[35] S.-Y. Park, S.-J. Yoon, S.-I. Hakomori, J.-M. Kim, J.-Y. Kim, B. Bernert, T. Ullman, S.H. Itzkowitz, J.H. Kim, Dimeric Lea (Lea-on-Lea) status of β-haptoglobin in sera of colon cancer, chronic inflammatory disease and normal subjects, Int. J. Oncol., 36 (2010) 1291-1297.
[36] R.S. Bresalier, J.C. Byrd, D. Tessler, J. Lebel, J. Koomen, D. Hawke, E. Half, K.F. Liu, N. Mazurek, C. Great Lakes-New England, A circulating ligand for galectin-3 glycoprotein elevated in individual is a haptoglobin-related with colon cancer, Gastroenterology, 127 (2004) 741-748.
[37] E. Saeland, A.I. Belo, S. Mongera, I. van Die, G.A. Meijer, Y. van Kooyk, Differential glycosylation of MUC1 and CEACAM5 between normal mucosa and tumour tissue of colon cancer patients, Int. J. Cancer, 131 (2012) 117-128.
[38] Y. Qiu, T.H. Patwa, L. Xu, K. Shedden, D.E. Misek, M. Tuck, G. Jin, M.T. Ruffin, D.K. Turgeon, S. Synal, Plasma glycoprotein profiling for colorectal cancer biomarker identification by lectin glycoarray and lectin blot, J. Proteome Res., 7 (2008) 1693-1703.
[39] C. Li, D.M. Simeone, D.E. Brenner, M.A. Anderson, K.A. Shedden, M.T. Ruffin, D.M. Lubman, Pancreatic cancer serum detection using a lectin/glyco-antibody array method, J. Proteome Res., 8 (2008) 483-492.
[40] J. Zhao, T.H. Patwa, W. Qiu, K. Shedden, R. Hinderer, D.E. Misek, M.A. Anderson, D.M. Simeone, D.M. Lubman, Glycoprotein microarrays with multi-lectin detection: unique lectin binding patterns as a tool for classifying normal, chronic pancreatitis and pancreatic cancer sera, J. Proteome Res., 6 (2007) 1864-1874.
[41] E. Miyoshi, M. Nakano, Fucosylated haptoglobin is a novel marker for pancreatic cancer: detailed analyses of oligosaccharide structures, Proteomics, 8 (2008) 3257-3262.
[42] S. Nie, A. Lo, J. Wu, J. Zhu, Z. Tan, D.M. Simeone, M.A. Anderson, K.A. Shedden, M.T. Ruffin, D.M. Lubman, Glycoprotein biomarker panel for pancreatic cancer discovered by quantitative proteomics analysis, J. Proteome Res., 13 (2014) 1873-1884.
[43] K. Kusama, Y. Okamoto, K. Saito, T. Kasahara, T. Murata, Y. Ueno, Y. Kobayashi, Y. Kamada, E. Miyoshi, Reevaluation of Pholiota squarrosa lectin-reactive haptoglobin as a pancreatic cancer biomarker using an improved ELISA system, Glycoconjugate J., (2017) 1-8.
[44] N. Parker, C. Makin, C. Ching, D. Eccleston, O. Taylor, D. Milton, J.M. Rhodes, A new enzyme-linked lectin/mucin antibody sandwich assay (CAM 17.1/WGA) assessed in combination with CA 19-9 and peanut lectin binding assay for the diagnosis of pancreatic cancer, Cancer, 70 (1992) 1062-1068.
[45] J.Y. Yiannakou, P. Newland, F. Calder, A.N. Kingsnorth, J.M. Rhodes, Prospective study of CAM 17 center dot 1/WGA mucin assay for serological diagnosis of pancreatic cancer, Lancet, 349 (1997) 389-392.
[46] T. Yue, I.J. Goldstein, M.A. Hollingsworth, K. Kaul, R.E. Brand, B.B. Haab, The prevalence and nature of glycan alterations on specific proteins in pancreatic cancer patients revealed using antibody-lectin sandwich arrays, Mol. Cel. Proteom., 8 (2009) 1697-1707.
[47] S. Pan, T.A. Brentnall, R. Chen, Glycoproteins and glycoproteomics in pancreatic cancer, World journal of gastroenterology, 22 (2016) 9288.
[48] K. Shimizu, K. Nakamura, S. Kobatake, S. Satomura, M. Maruyama, F. Kameko, J. Tajiri, R. Kato, The clinical utility of Lens culinaris agglutinin-reactive thyroglobulin ratio in serum for distinguishing benign from malignant conditions of the thyroid, Clin. Chim. Acta, 379 (2007) 101-104.
[49] T. Kanai, M. Amakawa, R. Kato, K. Shimizu, K. Nakamura, K.-i. Ito, Y. Hama, M. Fujimori, J. Amano, Evaluation of a new method for the diagnosis of alterations of Lens culinaris agglutinin binding of thyroglobulin molecules in thyroid carcinoma, Clinical chemistry and laboratory medicine, 47 (2009) 1285-1290.
[50] K. YAMAMOTO, T. TSUJI, O. TARUTANI, T. OSAWA, Structural changes of carbohydrate chains of human thyroglobulin accompanying malignant transformations of thyroid glands, The FEBS Journal, 143 (1984) 133-144.
[51] A. Naitoh, Y. Aoyagi, H. Asakura, Highly enhanced fucosylation of serum glycoproteins in patients with hepatocellular carcinoma, Journal of gastroenterology and hepatology, 14 (1999) 436-445.
[52] Y.H. Ahn, P.M. Shin, N.R. Oh, G.W. Park, H. Kim, J.S. Yoo, A lectin-coupled, targeted proteomic mass spectrometry (MRM MS) platform for identification of multiple liver cancer biomarkers in human plasma, J. Proteomics, 75 (2012) 5507-5515.
[53] Y.H. Ahn, P.M. Shin, Y.S. Kim, N.R. Oh, E.S. Ji, K.H. Kim, Y.J. Lee, S.H. Kim, J.S. Yoo, Quantitative analysis of aberrant protein glycosylation in liver cancer plasma by AAL-enrichment and MRM mass spectrometry, Analyst, 138 (2013) 6454-6462.
[54] J.H. Lee, C.H. Cho, S.H. Kim, J.G. Kang, J.S. Yoo, C.L. Chang, J.-H. Ko, Y.-S. Kim, Semiquantitative measurement of a specific glycoform using a DNA-tagged antibody and lectin affinity chromatography for glyco-biomarker development, Mol. Cel. Proteom., 14 (2015) 782-795.
[55] H. Toyoda, T. Kumada, T. Tada, Y. Kaneoka, A. Maeda, F. Kanke, S. Satomura, Clinical utility of highly sensitive Lens culinaris agglutinin-reactive alpha-fetoprotein in hepatocellular carcinoma patients with alpha-fetoprotein< 20 ng/mL, Cancer Sci., 102 (2011) 1025-1031.
[56] X. Yi, S. Yu, Y. Bao, Alpha-fetoprotein-L3 in hepatocellular carcinoma: a meta-analysis, Clin. Chim. Acta, 425 (2013) 212-220.
[57] M.A. Comunale, M. Wang, J. Hafner, J. Krakover, L. Rodemich, B. Kopenhaver, R.E. Long, O. Junaidi, A.M.D. Bisceglie, T.M. Block, Identification and development of fucosylated glycoproteins as biomarkers of primary hepatocellular carcinoma, J. Proteome Res., 8 (2008) 595-602.
[58] Y. Liu, J. He, C. Li, R. Benitez, S. Fu, J. Marrero, D.M. Lubman, Identification and Confirmation of Biomarkers Using an Integrated Platform for Quantitative Analysis of Glycoproteins and Their Glycosylations, J. Proteome Res., 9 (2010) 798-805.
[59] L.F. Hoagland, M.J. Campa, E.B. Gottlin, J.E. Herndon, E.F. Patz, Haptoglobin and posttranslational glycan-modified derivatives as serum biomarkers for the diagnosis of nonsmall cell lung cancer, Cancer, 110 (2007) 2260-2268.
[60] D. Wang, M. Hincapie, T. Rejtar, B.L. Karger, Ultrasensitive characterization of site-specific glycosylation of affinity-purified haptoglobin from lung cancer patient plasma using 10 µm id porous layer open tubular liquid chromatography- linear ion trap collision-induced dissociation/electron transfer dissociation mass spectrometry, Anal. Chem., 83 (2011) 2029-2037.
[61] H.Y. Tsai, K. Boonyapranai, S. Sriyam, C.J. Yu, S.W. Wu, K.H. Khoo, S. Phutrakul, S.T. Chen, Glycoproteomics analysis to identify a glycoform on haptoglobin associated with lung cancer, Proteomics, 11 (2011) 2162-2170.
[62] J.N. Arnold, R. Saldova, M.C. Galligan, T.B. Murphy, Y. Mimura-Kimura, J.E. Telford, A.K. Godwin, P.M. Rudd, Novel Glycan Biomarkers for the Detection of Lung Cancer, J. Proteome Res., 10 (2011) 1755-1764.
[63] Y. Hirao, H. Matsuzaki, J. Iwaki, A. Kuno, H. Kaji, T. Ohkura, A. Togayachi, M. Abe, M. Nomura, M. Noguchi, Glycoproteomics approach for identifying glycobiomarker candidate molecules for tissue type classification of non-small cell lung carcinoma, J. Proteome Res., 13 (2014) 4705-4716.
[64] M. Ferens-Sieczkowska, E.M. Kratz, B. Kossowska, E. Passowicz-Muszynska, R. Jankowska, Comparison of Haptoglobin and Alpha(1)-Acid Glycoprotein Glycosylation in the Sera of Small Cell and Non-Small Cell Lung Cancer Patients, Postep. Hig. Med. Dosw., 67 (2013) 828-836.
[65] Y.Q. Liang, T.R. Ma, A. Thakur, H.J. Yu, L. Gao, P.Y. Shi, X.T. Li, H. Ren, L.Y. Jia, S. Zhang, Z. Li, M.W. Chen, Differentially expressed glycosylated patterns of alpha-1-antitrypsin as serum biomarkers for the diagnosis of lung cancer, Glycobiology, 25 (2015) 331-340.
[66]S.D. Shiayan, V.V. Nasonov, N.V. Bovin, L.I. Novikova, V.A. Aleshkin, A.G. Lutov, STUDIES OF N-LINKED OLIGOSACCHARIDE CHAINS OF ALPHA(1)-ACID GLYCOPROTEIN ISOLATED FROM ASCITIC FLUID OF STOMACH-CANCER PATIENTS AND NORMAL SERUM, Eksperimentalnaya Onkologiya, 15 (1993) 53-61.
[67]J. Bones, J.C. Byrne, N. O'Donoghue, C. McManus, C. Scaife, H. Boissin, A. Nastase, P.M. Rudd, Glycomic and glycoproteomic analysis of serum from patients with stomach cancer reveals potential markers arising from host defense response mechanisms, J. Proteome Res., 10 (2010) 1246-1265.
[68]S.H. Lee, S. Jeong, J. Lee, I.S. Yeo, M.J. Oh, U. Kim, S. Kim, S.H. Kim, S.Y. Park, J.H. Kim, S.H. Park, J.H. Kim, H.J. An, Glycomic profiling of targeted serum haptoglobin for gastric cancer using nano LC/MS and LC/MS/MS, Mol. Biosyst., 12 (2016) 3611-3621.
[69]L. Valmu, H. Alfthan, K. Hotakainen, S. Birken, U.H. Stenman, Site-specific glycan analysis of human chorionic gonadotropin beta-subunit from malignancies and pregnancy by liquid chromatography-electrospray mass spectrometry, Glycobiology, 16 (2006) 1207-1218.
[70]T. Kamoto, S. Satomura, T. Yoshiki, Y. Okada, F. Henmi, H. Nishiyama, T. Kobayashi, A. Terai, T. Habuchi, O. Ogawa, Lectin-reactive alpha-fetoprotein (AFP-L3%) curability and prediction of clinical course after treatment of non-seminomatous germ cell tumors, Jpn. J. Clin. Oncol., 32 (2002) 472-476.
[71]S. Ambrose, N. Gordon, J. Goldsmith, W. Wei, M. Zeegers, N. James, M. Knowles, R. Bryan, D. Ward, Use of Aleuria alantia Lectin Affinity Chromatography to Enrich Candidate Biomarkers from the Urine of Patients with Bladder Cancer, Proteomes, 3 (2015) 266.
[72]R. Azevedo, A. Peixoto, C. Gaiteiro, E. Fernandes, M. Neves, L. Lima, L.L. Santos, J.A. Ferreira, Over forty years of bladder cancer glycobiology: Where do glycans stand facing precision oncology?, Oncotarget, 8 (2017) 91734-91764.
[73]E. Pocheć, A. Lityńska, M. Bubka, A. Amoresano, A. Casbarra, Characterization of the oligosaccharide component of α 3 β 1 integrin from human bladder carcinoma cell line T24 and its role in adhesion and migration, European journal of cell biology, 85 (2006) 47-57.
[74]S. Cotton, R. Azevedo, C. Gaiteiro, D. Ferreira, L. Lima, A. Peixoto, E. Fernandes, M. Neves, D. Neves, T. Amaro, R. Cruz, A. Tavares, M. Rangel, A.M.N. Silva, L.L. Santos, J.A. Ferreira, Targeted O-glycoproteomics explored increased sialylation and identified MUC16 as a poor prognosis biomarker in advanced-stage bladder tumours, Mol. Oncol., 11 (2017) 895-912.
[75]N. Yang, S. Feng, K. Shedden, X.L. Xie, Y.S. Liu, C.J. Rosser, D.M. Lubman, S. Goodison, Urinary Glycoprotein Biomarker Discovery for Bladder Cancer Detection Using LC/MS-MS and Label-Free Quantification, Clin. Cancer Res., 17 (2011) 3349-3359.
[76]Yamashita K, Umetsu K, Suzuki T, Ohkura T (1992) Purification and characterization of a Neu5Ac alpha 2-->6Gal beta 1-->4GIcNAc and HSO3(-)-->6Gal beta 1-->GlcNAc specific lectin in tuberous roots of Trichosanthes japonica. Biochemistry 31 (46):11647-11650.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

As used herein the terms "about", "approximately" or "essentially" mean within 20%, preferably within 15%, preferably within 10%, and more preferably within 5% of a given value or range. It also includes the concrete number, i.e. "about 20" includes the number of 20.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

### EXAMPLES

An even better understanding of the present invention and of its advantages will be evident from the following examples, offered for illustrative purposes only. The examples are not intended to limit the scope of the present invention in any way.

### Example 1

### Materials and methods

### Chemicals

Unconjugated streptavidin (~55 kDa, not glycosylated), MAA-II (~130 kDa) and anti-streptavidin antibody (~150 kDa) was purchased from Vector Labs, US. Glycan 3'-sialyllactosamine-PEG3-biotin (single arm, ~1100 Da) was obtained from Sussex Research, Canada. Desalting columns (MWCO = 7000 Da) were purchased from Thermo, US. All common chemicals, such as buffer components etc., were purchased from Sigma Merck, US. For all experiments, ultra pure deionized water (G = 0.055 µS) was used. All buffers were filtered prior to use using 0.22 µm sterile filters.

### Surface Plasmon Resonance (SPR)

All reagents used for SPR were purchased from GE Healthcare including HBS-P+ (Buffer 10×; BR-1006-71), Coupling Kit (BR100050), EDC (0.4 M), NHS (0.1 M), ethanolamine hydrochloride (1 M; pH 8.5). For regeneration NaOH (50 mM; BR-1003-58) and for coupling acetate buffer pH 4.0 (BR-1003-51) was used. SPR assays were run on BiacoreX100 (GE Healthcare) using a sensor chip CM5 (29-1496-04) or Au chip modified using 11-mercaptoundecanoic acid (5 mM in UV/VIS ethanol, Sigma Merck, US; incubated at RT overnight) under a constant flow rate of 30 µL/min at 25°C. Original SW Biacore X100 Control Software was used to run the instrument.

### Nanoparticle Tracking Analysis (NTA)

All samples were diluted in 0.1 M PB (pH 7.4, 0.22 µm filtered) and measured in continual flow (a total volume of 500 µl was prepared for a single run). Measurement concentrations were found by pretesting the ideal particle per frame value (20-100 particles/frame), as suggested. Following settings were set according to the manufacturer's software manual for NanoSight NS300: the detection threshold was determined to include as many particles as possible with the restrictions that 10-100 red crosses were counted. Blue cross count was limited to minimum. Autofocus was adjusted so that indistinct particles were avoided. For each measurement, five 60 s videos were captured under the following conditions: cell temperature: 25°C; syringe speed: 10 µl/min. After captures, the videos have been analyzed by the in-build NanoSight Software NTA 3.1 Build 3.1.46 with a detection threshold = 10.

### Results and discussion

### Streptavidin-glycan neoglycoprotein preparation

Lyophilized streptavidin powder was resuspended in a sterile 0.1 M PB (pH 7.4) to obtain a concentration of 1 mg.ml⁻¹. For known concentration of biotinylated glycan, these two solutions were mixed in 1+5 molecules ratio for an hour at 37°C with gentle mixing (500 rpm). Finally, this neoglycoprotein was purified of redundant glycans using Zeba Spin desalting column with 7k MWCO (previously equilibrated using PB) at 3000 rpm for 1 min. Neoglycoprotein was obtained at c = ~0.9 mg.ml⁻¹ and was stored at 4°C for the rest of the experimental work.

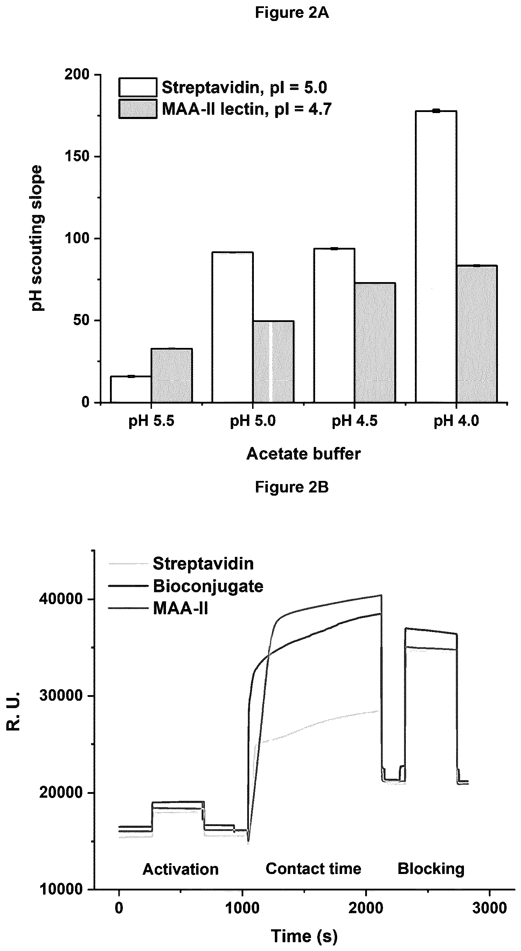

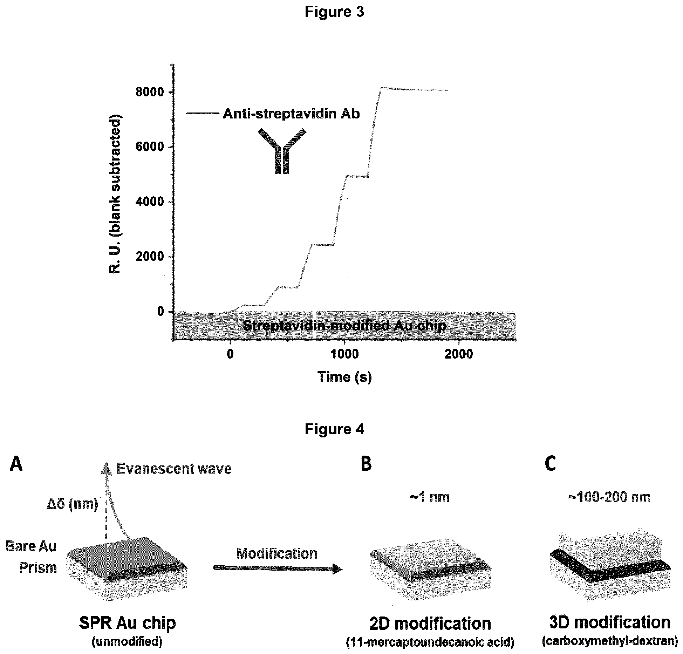

SPR binding analysis in the first run, a carboxymethyl-dextran CM5 SPR chip was used to find an optimal immobilization pH value and subsequently for immobilization of a ligand on the sensor surface. Optimal pH for all cases was 4.0 (**Fig 2A**) - based on the slope value during preconcentration of the ligand on sensor interface for different pH values. pl values for streptavidin and MAA-II lectin are 5.0 and 4.7, respectively, while similar value is predicted for neoglycoprotein as well. In all three cases, *i.e.* for streptavidin, neoglycoprotein and MAA-II lectin, amine coupling (EDC/NHS protocol) for activation and ethanolamine blocking was used (**Fig 2B**). To complete a sandwich configuration, anti-streptavidin antibody (Ab) and MAA-II lectin should bind to neoglycoprotein at the same time. However, probably due to sterical hindrance, quite high density of negative charge and layer thickness/distance from the prism/gold interface (and combination of these factors), only Ab binding to a streptavidin-modified CM5 chip during single cycle kinetics (SCK) could be observed (**Fig 3**). For the rest of experiments, the higher the concentration of a sample during SCK, usually the more negative response - even during lectin binding using neoglycoprotein-modified chip (with MAA-II, SNA-I and WGA, all of which should bind to sialylated/negatively charged glycan moieties).

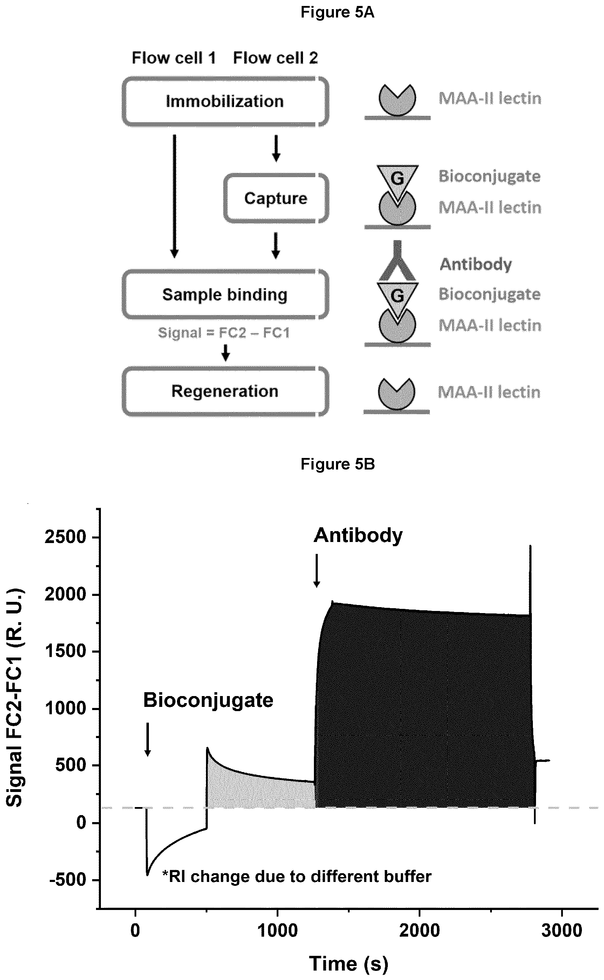

To confirm a successful preparation of a sandwich (MAA-II/glycan-streptavidin/antibody), a 2D chip was prepared by immersing bare Au chip into 5 mM ethanol solution of 11-mercaptoundecanoic acid (RT, dark, overnight). An advantage of this approach is that basically all negative charges (carboxy groups) are localized on the surface and are removed after immobilization/blocking. The whole sandwich preparation, as well as the assay workflow, is shown on **Fig 5A****.**

A detailed overview of the conditions applied for each step is summarized in following **Table 2.**

**Table 2: Overview of individual steps applied for sandwich preparation using SPR binding assay, i. e. type of molecule, conditions and results, as shown in Fig. 4. All R. U. are calculated after subtracting a blank (flow cell 1, FC1) from detection cell (FC2). Altough regeneration of the surface is optional and is not applied for Glycanostics MELLBA assay, the surface couldn't be completely regenerated using only 50 mM NaOH.**

| **Assay** | **Molecule** | **Conditions** | **Results / notes** |
|---|---|---|---|
| Immobilization | **MAA-II lectin** | Acetate buffer pH 4.0, amine coupling | FC1 = 433.8 R. U.; FC2 = 545.6 R. U. |
| Capture | **Neoglycoprotein** | Running buffer, c = 0.5 mg/ml, t = 420 s (600 s stabilization) | 224.5 R. U. |
| Sample | **Antibody** | Running buffer, c = 0.05 mg/ml, t = 120 s (1200 s stabilization) | 1457. 3 R. U. |
| Regeneration | - | NaOH, c = 50 mM, t = 30 s | (optional) |

### NTA analysis of MNPs modification and enrichment process

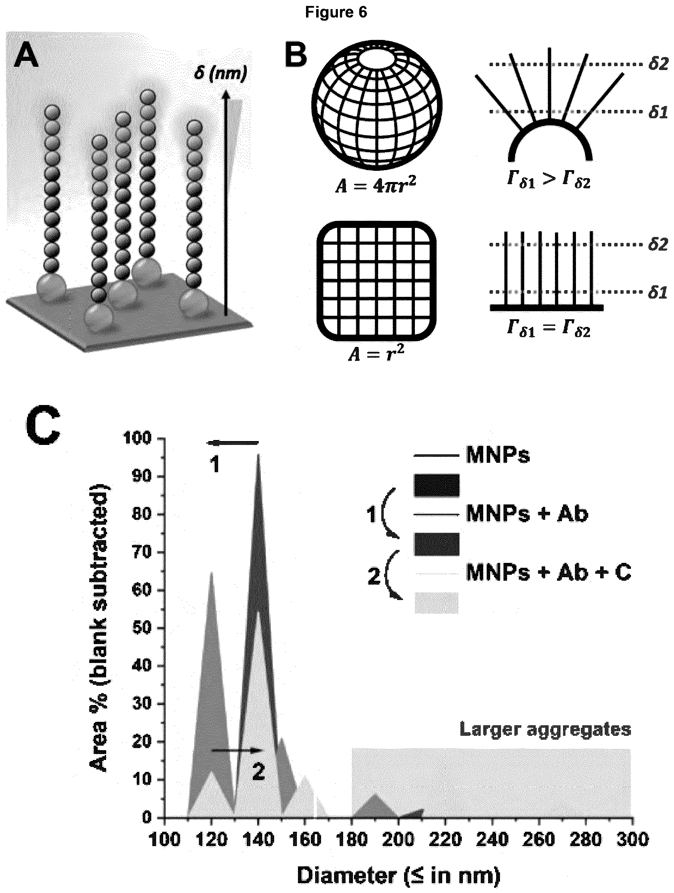

For NTA analysis to observe the modification of magnetic nanoparticles (MNPs, 130 nm COOH terminated, dextran-coated nanomags), all three samples, *i. e*. bare MNPs, Ab-modified MNPs MNPs + Ab) and neoglycoprotein-enriched Ab-modified MNPs (MNPs + Ab + C) were treated equally, whether using a specific chemical/component or PB in case of blank - all three samples were therefore separated the same way/same number of times. Using spherical interface for biorecognition yields higher signal in this case, since the ligand density is decreased with longer linker molecules, decreasing sterical hindrance for MELLBA compared to planar surface used for SPR experiments above (**Fig. 6A** and **B;** hypothesis, however published elsewhere). Results from NTA are shown in **Fig 6C****.** After modification/enrichment, modified MNPs were more likely to form sediment at the bottom of the test tube as a result of their increased diameter. For NTA analysis, each sample was diluted 500x in sterile PB.

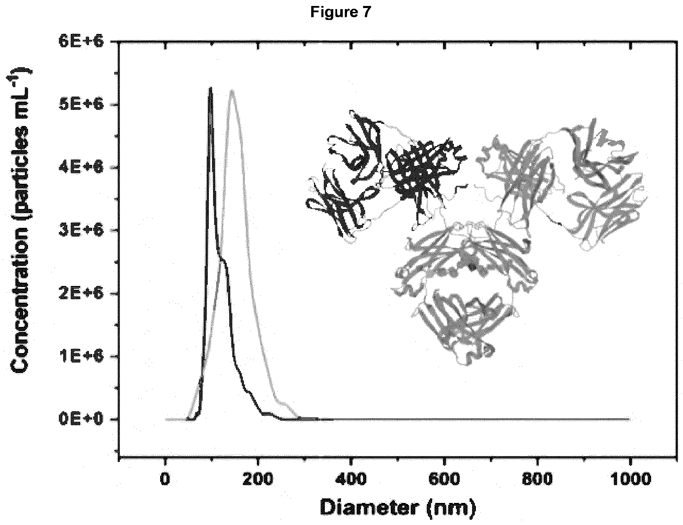

While unmodified (but equal number of times separated) MNPs yielded almost exclusively one single peak with d ≤ 140 nm, while after Ab conjugation (amine coupling, 10 min, RT) there are two major fractions - (i) ≤ 120 nm (slightly less compared to unmodified particles) and about 20% of (ii) ≤150 nm. After incubation of these particles with purified neoglycoprotein, at least four different fractions could be observed - even larger aggregates occurred at ~220 nm. Decrease of hydrodynamic diameter (*d*_{H}) in process 1 (ab immobilization) is caused by "compressing" of dextran matrix around immobilized ab in case the binding capacity of MNPs is not saturated during the immobilization process (hypothesis). Otherwise, *d*_{H} increases, as in **Fig 7****.** An important aspect of this experiment is the increase of *d*_{H} in process 2 by ~20 nm - a value affected by the fact the glycan (trisaccharide as well as PEG3 linker are heavily hydrated and quite flexible).

### MALDI-TOF MS and ELLBA to detect glycan content

Different streptavidin + biotinyl-glycan ratios were used (*i. e*. 1+0, 1+1, 1+2, 1+3, 1+4, 1+5, 1+6 and 1+7 ratios of number of molecules per volume) to prepare fully glycosylated protein standard. MALDI-TOF mass spectrometry (Bruker, USA) and previously optimized protocol (using 2,5-dihydroxyacetophenone (DHA) matrix) was used to detect mass fragments (m/z parameter) for ionized sample components, as described previously elsewhere. In **Fig. 9****,** mass spectra are shown in detail, namely streptavidin fragments in all samples (**A**), differing by ~13 kDa, a mass of one subunit in ~55 kDa homotetramer. The most intensive peak was always at ~13 kDa as well (**Fig. 8**), with the other peaks having much lower intensity. Only samples incubated previously with biotinyl-glycan (all except for 1+0, *i. e*. bare streptavidin, **Fig. 9B**) showed also presence of a peak with m/z = ~1300 Da, 3'-sialylated glycan derivative (**Fig. 9C**). Moreover, the intensity of this peak increased with increasing glycan/streptavidin ratio (**Fig. 9D**). However, since streptavidin can bind only up to 4 biotins, it is important to use a proper glycan/streptavidin ratio to maintain the full saturation of protein standard by these glycans.

For this purpose, enzyme-linked lectin binding assay (ELLBA) has been used to find the full saturation ratio. The assay configuration is depicted in **Fig. 9E** **left** - protein standard is immobilized on the bottom of the ELISA plate well, incubated with unconjugated MAA-II (2,3-Sialic acid/Gal specific, which can effectively block all the available glycan epitopes) and subsequently with biotinylated MAA-II, which bears several biotin molecules and is bound to free biotin-binding sites on unsaturated streptavidin standard. Subsequently, streptavidin-peroxidase is used to generate an optical signal (OPD/hydrogen peroxide). Streptavidin-HRP binds only to free biotin molecules present on biotinylated MAA-II, thus is in a reciprocal relation with the amount of glycan molecules present on streptavidin (**Fig. 9E** **right**).

### Conclusions

The neoglycoprotein (protein standard) made by attachment of biotinylated glycans to the streptavidin was simultaneously recognised by the MAA-II lectin and by the anti-streptavidin antibody. This unexpected finding shows that such a neoglycoprotein can be applied as a protein standard for ELISA-like formats of analysis (including MELLA).

### Example 2

Experimental work showed that a glycoprotein standard - also referred to herein as neoglycoprotein (standard) or standard of the present invention, e.g. as the standard applied in Example 1, above - is stable for at least 1 week when stored at 4°C. Such a standard can be prepared in a highly reproducible way i.e. RSD of 8.17% for preparation of glycoprotein standard in 17 independent preparation batches within 18 weeks.

## Claims

1. A method for relativizing a signal (1) obtained from determining a glycan structure (A) suspected to be present on a protein of interest, comprising
a) determining a glycan structure (A) suspected to be present on a protein of interest, which provides a signal (1); and
b) comparing the signal (1) obtained from determining said glycan structure (A) suspected to be present on said protein of interest in step a) with a signal (2) obtained from determining said glycan structure (A) actually comprised by a neoglycoprotein acting as a standard, wherein said neoglycoprotein comprises a streptavidin molecule bound through biotin-streptavidin interaction to at least one pre-defined glycan determinant which comprises said glycan structure (A),
wherein relativizing comprises comparing signal (1) with signal (2) from the standard, thereby signal (2) allows putting the information obtained by signal (1) in relation,
thereby relativizing said signal (1) to said signal (2), or vice versa.

2. The method of claim 1, wherein relativizing comprises
(i) if signal (1) is lower than signal (2), it is indicative that said suspected glycan structure (A) is not present on said protein of interest, or
(ii) if signal (1) is equal to or higher than signal (2), it is indicative that said suspected glycan structure (A) is present on said protein of interest.

3. The method of claim 1, wherein relativizing comprises
comparing the signal (1) obtained from determining said glycan structure (A) suspected to be present on said protein of interest with the signal (2) obtained from determining a concentration series of said glycan structure (A) actually comprised by said neoglycoprotein.

4. The method of claim 3, wherein the concentration series comprises a concentration which corresponds to a predetermined threshold concentration above which said glycan structure (A) is known to be present on said protein of interest.

5. Use of a neoglycoprotein acting as a standard comprising a streptavidin molecule bound through biotin to at least one pre-defined glycan determinant which actually comprises a glycan structure (A) suspected to be present on a protein of interest for relativizing a signal (1) obtained from determining glycan structure (A) suspected to be present on a protein of interest to a signal (2) obtained from determining said glycan structure (A) actually comprised by said neoglycoprotein, wherein said neoglycoprotein comprises a streptavidin molecule bound through biotin-streptavidin interaction to at least one pre-defined glycan determinant which comprises said glycan structure (A) wherein relativizing comprises comparing signal (1) with signal (2) from the standard, thereby signal (2) allows putting the information obtained by signal (1) in relation.

6. The use of claim 5, wherein relativizing comprises
(i) if signal (1) is lower than signal (2), it is indicative that said suspected glycan structure (A) is not present on said protein of interest, or
(ii) if signal (1) is equal to or higher than signal (2), it is indicative that said suspected glycan structure (A) is present on said protein of interest.

7. The use of claim 6, wherein relativizing comprises comparing the signal (1) obtained from determining said glycan structure (A) suspected to be present on said protein of interest with the signal (2) obtained from determining a concentration series of said glycan structure (A) actually comprised by said neoglycoprotein.

8. The use of claim 7, wherein the concentration series comprises a concentration which corresponds to a predetermined threshold concentration above which said glycan structure (A) is known to be present on said protein of interest.

9. The method of any one of claims 1-4 or the use of any one of claims 5-8, wherein the signal is signal intensity.

10. The method of any one of claims 1-4, and 9 or the use of any one of claims 5-9, wherein signal (1) and signal (2) are obtained by enzyme-linked immunosorbent assay (ELISA), enzyme-linked lectin assay (ELLA), magnetic ELLA (MELLA), preferably ELLA or MELLA.

11. The method of any one of claims 1-4, and 9-10 or the use of any one of claims 5-10, wherein the glycan structure (A) is selected from the group consisting of core fucose, antennary fucose, Fucα1-6GlcNAc-*N*-Asn containing N-linked oligosaccharides, Fucα1-6/3GlcNAc, α-L-Fuc, Fucα1-2Galβ1-4(Fucα1-3)GlcNAc, Fucα1-2Gal, Fucα1-6GlcNAc, Manβ1-4GlcNAcβ1-4GlcNAc, branched *N*-linked hexa-saccharide, Manα1-3Man, α-D-Man, (GlcNAcβ1-4)₂₋₄, Galβ1-4GlcNAc, GlcNAcα1-4Galβ1-4GlcNAc, (GlcNAcβ1-4)₂₋₅, Neu5Ac (sialic acid), Galβ1-3GalNAc-serine/threonine, Galα1-3GalNAc, Galβ1-6Gal, Galβ1-4GIcNAc, Galβ1-3GaINAc, GalNAcα1-3GalNAc, GalNAcα1-3Gal, GalNAcα/β1-3/4Gal, α-GalNAc, GalNAcβ1-4Gal, GalNAcα1-3(Fucα1-2)Gal, GalNAcα1-2Gal, GalNAcα1-3GalNAc, GalNAcβ1-3/4Gal, GalNAc-Ser/Thr (Tn antigen), Galβ1-3GalNAc-Ser/Thr (T antigen), GalNAcβ1-4GlcNAc (LacdiNAc), α-2,3Neu5Ac (α2-3 linked sialic acid), α-2,6Neu5Ac (α2-6 linked sialic acid), α-2,8Neu5Ac (α2-8 linked sialic acid), sialic acid (α-2,3Neu5Ac, α-2,6Neu5Ac or α-2,8Neu5Ac), Neu5Acα4/9-O-Ac-Neu5Ac, Neu5Acα2-3Galβ1-4Glc/GlcNAc, Neu5Acα2-6Gal/GalNAc, *N*-linked bi-antennary, *N*-linked tri/tetra-antennary, branched β1-6GIcNAc, Galα1-3(Fucα1-2)Galβ1-3/4GlcNAc, Galβ1-3(Fucα1-4)GlcNAc, NeuAcα2-3Galβ1-3(Fucα1-4)GlcNAc, Fucα1-2Galβ1-3(Fucα1-4)GlcNAc, Galβ1-4(Fucα1-3)GlcNAc, NeuAcα2-3Galβ1-4(Fucα1-3)GlcNAc, Fucα1-2Galβ1-4(Fucα1-3)GlcNAc, high mannose, sialyl Lewis^{a} (sialyl Le^{a}) antigen, sialyl Lewis^{x} (sialyl Le^{x}) antigen, Lewis^{x} (Le^{x}) antigen, sialyl Tn antigen, sialyl T antigen, Lewis^{y} (Le^{y}) antigen, sulfated core1 glycan, Tn antigen, T antigen, core 2 glycan, Lewis^{a} (Le^{a}) antigen, (GlcNAcβ1-4)ₙ, β-D-GlcNAc, GalNAc, Gal-GlcNAc, GlcNAc, Galα1-3Gal, Galβ1-3GalNAc, α-Gal, α-GalNAc, (GlcNAc)ₙ, branched (LacNAc)ₙ.

12. The method of any one of claims 1-4, and 9-11 or the use of any one of claims 5-11, wherein the protein of interest is a cancer biomarker protein, an autoimmune disease biomarker protein or an inflammatory disease biomarker protein.

13. The method or the use of claim 12, wherein said cancer biomarker protein is an ovarian cancer biomarker protein, a breast cancer biomarker protein, a colorectal cancer biomarker protein, a pancreatic cancer biomarker protein, a prostate cancer biomarker protein, a thyroid cancer biomarker protein, a liver cancer biomarker protein, a lung cancer biomarker protein, a stomach cancer biomarker protein, a testicular cancer biomarker protein or a bladder cancer biomarker protein.

14. The method or the use of claim 12, wherein said prostate cancer biomarker protein is β-haptoglobin, TIMP-1, PSA, fPSA or tPSA.

15. The method of any one of claims 1-4, and 9-14 or the use of any one of claims 5-14, wherein presence of said glycan structure (A) is indicative of cancer.

## Patentansprüche

1. Verfahren zum Relativieren eines Signals (1), erhalten durch das Bestimmen einer Glykanstruktur (A), die auf einem Protein von Interesse vorhanden sein könnte, umfassend:
a) Bestimmen einer Glykanstruktur (A), die auf einem Protein von Interesse vorhanden sein könnte, welches ein Signal (1) liefert; und
b) Vergleichen des Signals (1) erhalten durch das Bestimmen der Glykanstruktur (A), die auf dem Protein von Interesse in Schritt a) vorhanden sein könnte, mit einem Signal (2) erhalten durch das Bestimmen der Glykanstruktur (A), die tatsächlich von einem Neoglykoprotein umfasst ist, das als Standard dient, wobei das Neoglykoprotein ein Streptavidin-Molekül gebunden durch Biotin-Streptavidin-Wechselwirkung an mindestens eine vordefinierte Glykandeterminante umfasst, die die Glykanstruktur (A) umfasst,
wobei Relativieren Vergleichen von Signal (1) zu Signal (2) des Standards umfasst, wodurch es Signal (2) ermöglicht, die Informationen erhalten durch Signal (1) in Relation zu setzen, wodurch das Signal (1) zu dem Signal (2) relativiert wird, oder umgekehrt.

2. Verfahren nach Anspruch 1, wobei das Relativieren umfasst:
(i) falls Signal (1) niedriger ist als Signal (2), ist dies ein Hinweis darauf, dass die vermutete Glykanstruktur (A) nicht auf dem Protein von Interesse vorhanden ist, oder
(ii) falls Signal (1) gleich oder höher als Signal (2) ist, ist dies ein Hinweis darauf, dass die vermutete Glykanstruktur (A) auf dem Protein von Interesse vorhanden ist.

3. Verfahren nach Anspruch 1, wobei das Relativieren umfasst
Vergleichen des Signals (1), erhalten durch das Bestimmen der Glykanstruktur (A), die auf dem Protein von Interesse vorhanden sein könnte, mit dem Signal (2), erhalten durch das Bestimmen einer Konzentrationsreihe der Glykanstruktur (A), die tatsächlich von dem Neoglykoprotein umfasst ist.

4. Verfahren nach Anspruch 3, wobei die Konzentrationsreihe eine Konzentration umfasst, die einer vorbestimmten Schwellenkonzentration entspricht, oberhalb derer die Glykanstruktur (A) bekanntermaßen auf dem Protein von Interesse vorhanden ist.

5. Verwendung eines Neoglykoproteins als Standard umfassend ein Streptavidin-Molekül gebunden durch Biotin an mindestens eine vordefinierte Glykandeterminante, die tatsächlich eine Glykanstruktur (A) umfasst, die auf einem Protein von Interesse vorhanden sein könnte, zum Relativieren eines Signals (1), erhalten durch das Bestimmen der Glykanstruktur (A), die auf einem Protein von Interesse vorhanden sein könnte, zu einem Signal (2), erhalten durch das Bestimmen der Glykanstruktur (A), die tatsächlich von dem Neoglykoprotein umfasst ist, wobei das Neoglykoprotein ein Streptavidin-Molekül gebunden durch Biotin-Streptavidin-Wechselwirkung an mindestens eine vordefinierte Glykandeterminante umfasst, die die Glykanstruktur (A) umfasst wodurch Relativieren Vergleichen von Signal (1) mit Signal (2) des Standards umfasst, wodurch es Signal (2) ermöglicht, die Informationen erhalten durch Signal (1) in Relation zu setzen.

6. Verwendung nach Anspruch 5, wobei das Relativieren umfasst:
(i) falls Signal (1) niedriger ist als Signal (2), ist dies ein Hinweis darauf, dass die vermutete Glykanstruktur (A) nicht auf dem Protein von Interesse vorhanden ist, oder
(ii) falls Signal (1) gleich oder höher als Signal (2) ist, ist dies ein Hinweis darauf, dass die vermutete Glykanstruktur (A) auf dem Protein von Interesse vorhanden ist.

7. Verwendung nach Anspruch 6, wobei das Relativieren umfasst Vergleichen des Signals (1), erhalten durch das Bestimmen der Glykanstruktur (A), die auf dem Protein von Interesse vorhanden sein könnte, mit dem Signal (2), erhalten durch das Bestimmen einer Konzentrationsreihe der Glykanstruktur (A), die tatsächlich von dem Neoglykoprotein umfasst ist.

8. Verwendung nach Anspruch 7, wobei die Konzentrationsreihe eine Konzentration umfasst, die einer vorbestimmten Schwellenkonzentration entspricht, oberhalb derer die Glykanstruktur (A) bekanntermaßen auf dem Protein von Interesse vorhanden ist.

9. Verfahren nach einem der Ansprüche 1-4 oder Verwendung nach einem der Ansprüche 5-8, wobei das Signal Signalintensität ist.

10. Verfahren nach einem der Ansprüche 1-4, und 9 oder Verwendung nach einem der Ansprüche 5-9, wobei Signal (1) und Signal (2) erhalten sind durch Enzymgebundener Immunosorbent-Assay (ELISA), Enzymgebundener Lektin-Assay (ELLA), Magnetischer ELLA (MELLA), vorzugsweise ELLA oder MELLA.

11. Verfahren nach einem der Ansprüche 1-4, und 9-10 oder Verwendung nach einem der Ansprüche 5-10, wobei die Glykanstruktur (A) ausgewählt ist aus der Gruppe bestehend aus Kernfukose, Antennenfukose, Fucα1-6GlcNAc-*N*-Asn enthaltende N-verknüpfte Oligosaccharide, Fucα1-6/3GlcNAc, α-L-Fuc, Fucα1-2Galβ1-4(Fucα1-3)GlcNAc, Fucα1-2Gal, Fucα1-6GlcNAc, Manβ1-4GlcNAcβ1-4GlcNAc, verzweigtes N-verknüpftes Hexasaccharid, Manα1-3Man, α-D-Man, (GlcNAcβ1-4)₂₋₄, Galβ1-4GlcNAc, GlcNAcα1-4Galβ1-4GlcNAc, (GlcNAcβ1-4)₂₋₅, Neu5Ac (Sialinsäure), Galβ1-3GalNAc-Serin/Threonin, Galα1-3GalNAc, Galβ1-6Gal, Galβ1-4GlcNAc, Galβ1-3GalNAc, GalNAcα1-3GalNAc, GalNAcα1-3Gal, GalNAcα/β1-3/4Gal, α-GalNAc, GalNAcβ1-4Gal, GalNAcα1-3(Fucα1-2)Gal, GalNAcα1-2Gal, GalNAcα1-3GalNAc, GalNAcβ1-3/4Gal, GalNAc-Ser/Thr (Tn Antigen), Galβ1-3GalNAc-Ser/Thr (T Antigen), GalNAcβ1-4GlcNAc (LacdiNAc), α-2,3Neu5Ac (α2-3 verknüpfte Sialinsäure), α-2,6Neu5Ac (α2-6 verknüpfte Sialinsäure), α-2,8Neu5Ac (α2-8 verknüpfte Sialinsäure), Sialinsäure (α-2,3Neu5Ac, α-2,6Neu5Ac oder α-2,8Neu5Ac), Neu5Acα4/9-O-Ac-Neu5Ac, Neu5Aca2-3Galβ1-4Glc/GlcNAc, Neu5Acα2-6Gal/GalNAc, *N*-verknüpftes zweiantenniges, N-verknüpftes drei-/vierantenniges, verzweigtes β1-6GlcNAc, Galα1-3(Fucα1-2)Galβ1-3/4GlcNAc, Galβ1-3(Fucα1-4)GlcNAc, NeuAcα2-3Galβ1-3(Fucα1-4)GlcNAc, Fucα1-2Galβ1-3(Fucα1-4)GlcNAc, Galβ1-4(Fucα1-3)GlcNAc, NeuAcα2-3Galβ1-4(Fucα1-3)GlcNAc, Fucα1-2Galβ1-4(Fucα1-3)GlcNAc, High-Mannose, Sialyl-Lewis^{a} (Sialyl-Le^{a})-Antigen, Sialyl-Lewis^{x} (Sialyl-Le^{x})-Antigen, Lewis^{x} (Le^{x})-Antigen, Sialyl-Tn-Antigen, Sialyl-T-Antigen, Lewis^{y} (Le^{y})-Antigen, sulfatiertes Core-1-Glykan, Tn-Antigen, T-Antigen, Core-2-Glykan, Lewis^{a} (Le^{a})-Antigen, (GlcNAcβ1-4)ₙ, β-D-GlcNAc, GalNAc, Gal-GlcNAc, GlcNAc, Galα1-3Gal, Galβ1-3GalNAc, α-Gal, α-GalNAc, (GlcNAc)ₙ, verzweigtes (LacNAc)ₙ.

12. Verfahren nach einem der Ansprüche 1-4, und 9-11 oder Verwendung nach einem der Ansprüche 5-11, wobei das Protein von Interesse ein Krebs-Biomarker-Protein, ein Autoimmunerkrankungs-Biomarker-Protein oder ein Entzündungskrankheits-Biomarker-Protein ist.

13. Verfahren oder Verwendung nach Anspruch 12, wobei das Krebs-Biomarker-Protein ein Eierstockkrebs-Biomarker-Protein, ein Brustkrebs-Biomarker-Protein, ein Kolorektalkrebs-Biomarker-Protein, ein Bauchspeicheldrüsenkrebs-Biomarker-Protein, ein Prostatakrebs-Biomarker-Protein, ein Schilddrüsenkrebs-Biomarker-Protein, ein Leberkrebs-Biomarker-Protein, ein Lungenkrebs-Biomarker-Protein, ein Magenkrebs-Biomarker-Protein, ein Hodenkrebs-Biomarker-Protein oder ein Blasenkrebs-Biomarker-Protein ist.

14. Verfahren oder Verwendung nach Anspruch 12, wobei das Prostatakrebs-Biomarker-Protein Haptoglobin, TIMP-1, PSA, fPSA oder tPSA ist.

15. Verfahren nach einem der Ansprüche 1-4, und 9-14 oder Verwendung nach einem der Ansprüche 5-14, wobei das Vorhandensein der Glykanstruktur (A) ein Hinweis auf Krebs ist.

## Revendications

1. Procédé pour relativiser un signal (1), obtenu par la détermination d'une structure de glycanes (A) qui pourrait être présente sur une protéine d'intérêt, comprenant :
a) la détermination d'une structure de glycanes (A) qui pourrait être présente sur une protéine d'intérêt, laquelle fournit un signal (1); et
b) la comparaison du signal (1) obtenu par la détermination de la structure de glycanes (A) qui pourrait être présente sur la protéine d'intérêt dans l'étape a) avec un signal (2) obtenu par la détermination de la structure de glycanes (A) réellement présente dans une néoglycoprotéine servant de référence, ladite néoglycoprotéine comprenant une molécule de streptavidine liée par interaction biotine-streptavidine à au moins un déterminant de glycanes prédéfini qui comprend la structure de glycanes (A),
dans lequel la relativisation consiste à comparer le signal (1) avec le signal (2) du standard, ce qui permet au signal (2) de mettre en relation les informations obtenues par le signal (1), relativisant ainsi le signal (1) par rapport au signal (2), ou inversement.

2. Procédé selon la revendication 1, dans lequel la relativisation consiste à:
(i) si le signal (1) est inférieur au signal (2), cela indique que la structure de glycanes (A) supposée n'est pas présente sur la protéine d'intérêt, ou
(ii) si le signal (1) est égal ou supérieur au signal (2), cela indique que la structure de glycanes (A) supposée est présente sur la protéine d'intérêt.

3. Procédé selon la revendication 1, dans lequel la relativisation consiste à:
comparer le signal (1), obtenu par la détermination de la structure de glycanes (A) qui pourrait être présente sur la protéine d'intérêt, avec le signal (2), obtenu par la détermination d'une série de concentrations de la structure de glycanes (A) réellement présente dans la néoglycoprotéine.

4. Procédé selon la revendication 3, dans lequel la série de concentrations comprend une concentration correspondant à une concentration seuil prédéfinie, au-delà de laquelle la structure de glycanes (A) est connue pour être présente sur la protéine d'intérêt.

5. Utilisation d'une néoglycoprotéine servant de référence comprenant une molécule de streptavidine liée par biotine à au moins un déterminant de glycanes prédéfini qui comprend réellement une structure de glycanes (A) supposée présente sur une protéine d'intérêt, pour relativiser un signal (1) obtenu par la détermination de la structure de glycanes (A) supposée présente sur une protéine d'intérêt à un signal (2) obtenu par la détermination de la structure de glycanes (A) réellement présente dans la néoglycoprotéine, ladite néoglycoprotéine comprenant une molécule de streptavidine liée par interaction biotine-streptavidine à au moins un déterminant de glycanes prédéfini qui comprend la structure de glycanes (A), la relativisation consistant à comparer le signal (1) avec le signal (2) du standard, ce qui permet au signal (2) de mettre en relation les informations obtenues par le signal (1).

6. Utilisation selon la revendication 5, dans laquelle la relativisation consiste à:
(i) si le signal (1) est inférieur au signal (2), cela indique que la structure de glycanes ( A) supposée n'est pas présente sur la protéine d'intérêt, ou
(ii) si le signal (1) est égal ou supérieur au signal (2), cela indique que la structure de glycanes (A) supposée est présente sur la protéine d'intérêt.

7. Utilisation selon la revendication 6, dans laquelle la relativisation consiste à comparer le signal (1), obtenu par la détermination de la structure de glycanes (A) qui pourrait être présente sur la protéine d'intérêt, avec le signal (2), obtenu par la détermination d'une série de concentrations de la structure de glycanes (A) réellement présente dans la néoglycoprotéine.

8. Utilisation selon la revendication 7, dans laquelle la série de concentrations comprend une concentration correspondant à une concentration seuil prédéfinie, au-delà de laquelle la structure de glycanes (A) est connue pour être présente sur la protéine d'intérêt.

9. Procédé selon l'une des revendications 1-4 ou utilisation selon l'une des revendications 5-8, où le signal est l'intensité du signal.

10. Procédé selon l'une des revendications 1-4, et 9 ou utilisation selon l'une des revendications 5-9, dans lequel le signal (1) et le signal (2) sont obtenus par test immuno-enzymatique lié à un support (ELISA), test de lectine lié à un support (ELLA), test magnétique ELLA (MELLA), de préférence ELLA ou MELLA.

11. Procédé selon l'une des revendications 1 à 4 et 9 à 10 ou utilisation selon l'une des revendications 5 à 10, la structure glycanique (A) étant choisie dans le groupe constitué par le fucose central, le fucose antennaire, Oligosaccharides liés à l'azote contenant Fucα1-6GlcNAc-N-Asn, Fucα1-6/3GlcNAc, α-L-Fuc, Fucα1-2Galβ1-4(Fucα1-3)GlcNAc, Fucα1-2Gal, Fucα1-6GlcNAc, Manβ1-4GlcNAcβ1-4GlcNAc, hexasaccharide ramifié lié à l'azote, Manα1-3Man, α-D-Man, (GlcNAcβ1-4)₂₋₄, Galβ1-4GlcNAc, GlcNAcα1-4Galβ1-4GlcNAc, (GlcNAcβ1-4)₂₋₅, Neu5Ac (acide sialique), Galβ1-3GalNAc- Sérine/thréonine, Galα1-3GalNAc, Galβ1-6Gal, Galβ1-4GlcNAc, Galβ1-3GalNAc, GalNAcα1-3GalNAc, GalNAcα1-3Gal, GalNAcα/β1-3/4Gal, α-GalNAc, GalNAcβ1-4Gal, GalNAcα1-3(Fucα1-2)Gal, GalNAcα1-2Gal, GalNAcα1-3GalNAc, GalNAcβ1-3/4Gal, GalNAc-Ser/Thr (Tn antigène), Galβ1-3GalNAc-Ser/Thr (T antigène), GalNAcβ1-4GlcNAc (LacdiNAc), α-2,3Neu5Ac (α2-3 acide sialique lié), α-2,6Neu5Ac (α2-6 acide sialique lié), α-2,8Neu5Ac (α2-8 acide sialique lié), acide sialique (α-2,3Neu5Ac, α-2,6Neu5Ac oder α-2,8Neu5Ac), Neu5Acα4/9-O-Ac-Neu5Ac, Neu5Acα2-3Galβ1-4Glc/GlcNAc, Neu5Acα2-6Gal/GalNAc, N-lié à deux antennes, N-lié à trois/quatre antennes, ramifié β1-6GlcNAc, Galα1-3(Fucα1-2)Galβ1-3/4GlcNAc, Galβ1-3(Fucα1-4)GlcNAc, NeuAcα2-3Galβ1-3(Fucα1-4)GlcNAc, Fucα1-2Galβ1-3(Fucα1-4)GlcNAc, Galβ1-4(Fucα1-3)GlcNAc, NeuAcα2-3Galβ1-4(Fucα1-3)GlcNAc, Fucα1-2Galβ1-4(Fucα1-3)GlcNAc, riche en mannose, antigène sialyl-Lewis^{a} (sialyl-Le^{a}), antigène sialyl-Lewis^{x} (sialyl-Le^{x}), antigène Lewis^{x} (Le^{x}), antigène sialyl-Tn, antigène sialyl-T, antigène Lewis^{y} (Le^{y}), glycan Core-1 sulfaté, antigène Tn, antigène T, glycan Core-2, antigène Lewis^{a} (Le^{a}), (GlcNAcβ1-4)ₙ, β-D-GlcNAc, GalNAc, Gal-GlcNAc, GlcNAc, Galα1-3Gal, Galβ1-3GalNAc, α-Gal, α-GalNAc, (GlcNAc)ₙ, ramifié (LacNAc)ₙ.

12. Procédé selon l'une des revendications 1-4, et 9-11 ou utilisation selon l'une des revendications 5-11, dans lequel la protéine d'intérêt est une protéine biomarqueur de cancer, une protéine biomarqueur de maladie auto-immune ou une protéine biomarqueur de maladie inflammatoire.

13. Procédé ou utilisation selon la revendication 12, dans lequel la protéine biomarqueur de cancer est une protéine biomarqueur de cancer de l'ovaire, une protéine biomarqueur de cancer du sein, une protéine biomarqueur de cancer colorectal, une protéine biomarqueur de cancer du pancréas, une protéine biomarqueur de cancer de la prostate, une protéine biomarqueur de cancer de la thyroïde, une protéine biomarqueur de cancer du foie, une protéine biomarqueur de cancer du poumon, une protéine biomarqueur de cancer de l'estomac, une protéine biomarqueur de cancer des testicules ou une protéine biomarqueur de cancer de la vessie.

14. Procédé ou utilisation selon la revendication 12, dans lequel la protéine biomarqueur de cancer de la prostate est l'haptoglobine, TIMP-1, PSA, fPSA ou tPSA.

15. Procédé selon l'une des revendications 1-4, et 9-14 ou utilisation selon l'une des revendications 5-14, dans lequel la présence de la structure de glycanes (A) est un indice de cancer.
